# EUROPÄISCHE PATENTSCHRIFT

(11) **EP 2 390 310 B1**
(45) Veröffentlichungstag und Bekanntmachung des Hinweises auf die Patenterteilung: **23.11.2016**
(21) Anmeldenummer: 10164296.5
(22) Anmeldetag: 28.05.2010
(51) Int. Cl.: C12N 5/00

(54) **Thermoresponsives Multischichtsubstrat für biologische Zellen**
Thermoresponsive multilayer substrate for biological cells
Substrat multicouche thermosensible pour cellules biologiques

(43) Veröffentlichungstag der Anmeldung: 30.11.2011
(73) Patentinhaber: Fraunhofer-Gesellschaft zur Förderung der angewandten Forschung e.V., 80686 München (DE)
(72) Erfinder: Wischerhoff, Erik, 14469 Potsdam (DE); Lutz, Jean-Francois, 14467 Potsdam (DE); Laschewsky, André, 14469 Potsdam (DE); Duschl, Claus, 10115 Berlin (DE); Lankenau, Andreas, 14469 Potsdam (DE); Schmidt, Stephan, 10963 Berlin (DE); Badi, Nezha, 14467 Potsdam (DE)
(74) Vertreter: Haggenmüller, Christian

(56) Entgegenhaltungen:
- NOLAN C M ET AL: "Phase transition behavior, protein adsorption, and cell adhesion resistance of poly(ethylene glycol) cross-linked microgel particles" BIOMACROMOLECULES JULY/AUGUST 2005 AMERICAN CHEMICAL SOCIETY US, Bd. 6, Nr. 4, Juli 2005 (2005-07), Seiten 2032-2039, XP002604259 DOI: DOI:10.1021/BM0500087
- NAGASE K ET AL: "Temperature-responsive intelligent interfaces for biomolecular separation and cell sheet engineering" JOURNAL OF THE ROYAL SOCIETY INTERFACE 20090606 ROYAL SOCIETY OF LONDON GBR, Bd. 6, Nr. SUPPL. 3, 6. Juni 2009 (2009-06-06), Seiten S293-S309, XP002604260 DOI: DOI:10.1098/RSIF.2008.0499.FOCUS
- LYNCH I ET AL: "Novel method to prepare morphologically rich polymeric surfaces for biomedical applications via phase separation and arrest of microgel particles" JOURNAL OF PHYSICAL CHEMISTRY B 20060803 AMERICAN CHEMICAL SOCIETY US, Bd. 110, Nr. 30, 3. August 2006 (2006-08-03), Seiten 14581-14589, XP002604261 DOI: DOI:10.1021/JP061166A
- SCHMIDT S. ET AL.: "Adhesion and Mechanical Properties of PNIPAM Microgel Films and Their Potential Use as Switchable Cell Culture Substrates" ADVANCED FUNCTIONAL MATERIALS, [Online] Bd. 20, Nr. 19, 4. August 2010 (2010-08-04), Seiten 3235-3243, XP002604262 DOI: 10.1002/adfm.201000730 Gefunden im Internet: URL:http://onlinelibrary.wiley.com/doi/10. 1002/adfm.201000730/abstract;jsessionid=AF 1F60003B6135D9BD48C5FDE50FF1B3.d02t01> [gefunden am 2010-10-08]

## Beschreibung

Die Erfindung betrifft ein aus einem mehrlagigen Schichtsystem aufgebautes thermoresponsives Substrat zur Aufnahme biologischer Zellen, insbesondere ein Substrat, dessen Oberflächeneigenschaften in Abhängigkeit von der Temperatur veränderlich sind. Des Weiteren betrifft die Erfindung ein Verfahren zur Herstellung eines derartigen Substrats, insbesondere ein Verfahren zur Aufbringung eines erfindungsgemäßen Schichtsystems auf einen Substratkörper. Des Weiteren betrifft die Erfindung ein Verfahren zur Kultivierung biologischer Zellen auf einem thermoresponsiven Substrat. Anwendungen der Erfindung sind bei der *in-vitro-*Kultivierung biologischer Zellen gegeben.

Es ist allgemein bekannt, lebende biologische Zellen auf Substraten außerhalb eines Organismus zu kultivieren (*in*-*vitro*-Kultivierung). Ein zur Kultivierung vorgesehenes Substrat (Kultivierungssubstrat)besitzt typischerweise einen festen Substratkörper, z.B. aus Glas oder Kunststoff, dessen Oberfläche (Trägerfläche) funktionalisiert ist. Mit der Funktionalisierung, die z.B. eine Plasmabehandlung, eine Beschichtung mit Proteinen (wie z.B. Fibronektin, Kollagen) oder eine Beschichtung mit Polymeren (wie z.B. Polylysin) umfasst, wird eine Wechselwirkung der Zellen mit der Oberfläche beeinflusst. Es besteht ein Interesse an Kultivierungssubstraten, die eine gezielte Manipulation der biologischen Zellen und insbesondere eine Beeinflussung z.B. der Adhäsion, Migration, Proliferation, Differenzierung oder Zelltransformation (Bildung von Tumorzellen) ermöglichen. Ein ganz grundsätzliches Problem stellt die schonende Ablösung der Zellen von den Oberflächen dar. Dies geschieht in der Regel über eine Enzymbehandlung (Trypsinierung), die zu Zellschädigung und Zellverlusten führt.

Durch Experimente wurde festgestellt, dass Eigenschaften biologischer Zellen durch die Härte der Oberfläche des Kultivierungssubstrats beeinflusst werden können. Beispielsweise führten Härtevariationen, die durch sequenzielle Beschichtungen mit Polyacrylamid und Biomolekülen erzielt wurden, zu verschiedenen Differenzierungen von mesenchymalen Stammzellen (siehe Discher et al., Cell 126 (2006) 677-689). Des Weiteren ist bekannt, dass die Adhäsion biologischer Zellen von der Härte der Substratoberfläche abhängig ist. Es wurde festgestellt, dass die Adhäsion auf Substraten, die mit dem thermoresponsiven Polymer Poly-(N-isopropyl-acrylamid) ("PNIPam") oder Derivaten davon beschichtet sind und eine temperaturabhängige Hydratisierung aufweisen, gezielt in Abhängigkeit von der Temperatur beeinflusst werden kann (siehe N. Yamada et al., Makromol. Chem. 11 (1990) 571; C. Williams et al., Adv. Mater. 21 (2009) 2161-2164, O. Ernst et al., Lab Chip 7 (2007) 1322.). Diese Eigenschaft wurde vor kurzem auch an Polyethylenglykol (PEG)-basierten Polymeren gezeigt (siehe E. Wischerhoff et al. Angew. Chem. 47 (2008) 5666)

Herkömmliche Substrate, deren Oberflächen mit thermoresponsiven Polymeren beschichtet sind, können sowohl in Bezug auf die Herstellung der Beschichtung als auch in Bezug auf die Eignung für die Zellkultivierung Nachteile haben. So erfordert die Herstellung eines mit einem thermoresponsiven Polymer beschichteten Substrats mehrere aufwendige Prozessschritte, die mit einem kostspieligen apparativen Aufbau realisiert werden. Des Weiteren besteht nur eine beschränkte Variabilität der Polymerzusammensetzung. Beispielsweise kann das thermische Ansprechverhalten des thermoresponsiven Polymers sich verändern oder verschwinden, wenn dem Polymer eine zweite Polymerkomponente zugesetzt wird. Daher besteht nur eine beschränkte Flexibilität hinsichtlich der Einführung einer weiteren Funktionalisierung eines mit einem thermoresponsiven Polymer beschichteten Substrats.

Zur Herstellung von mit thermoresponsiven Polymeren beschichteten Substraten wurden in der Praxis verschiedene Protokolle zur Funktionalisierung des Substratkörpers entwickelt, wie z.B. Reaktionen mit Silanen, eine Plasmabehandlung oder eine chemische Funktionalisierung. Dabei werden an der Oberfläche des Substratkörpers funktionelle Gruppen, wie z. B. -NH₂, -COOH oder Epoxide bereitgestellt, die komplementär funktionalisierten Molekülen, insbesondere geeignet funktionalisierten thermoresponsiven Polymeren, eine kovalente Anbindung ermöglichen. Dabei hat sich eine beschränkte Reproduzierbarkeit und Steuerbarkeit der Funktionalisierung insbesondere in Bezug auf die Anbindungsdichte und Homogenität als nachteilig erwiesen. Die Herstellung einer Oberfläche mit definierten Mischungen verschiedener Moleküle ist nur in speziellen Fällen möglich.

Für die Kultivierung biologischer Zellen hat sich insbesondere die folgende Eigenschaft thermoresponsiver Polymere als nachteilig erwiesen. Allgemein ist ein thermoresponsives Polymer ein Polymer, das in Abhängigkeit von der Temperatur einen physikalischen Phasenübergang durchläuft, bei dem z.B. eine Umordnung von Polymerketten erfolgt. Während der Phasenübergang in einer Lösung in einem Temperaturbereich von wenigen °C scharf definiert ist, zeichnen sich schichtförmig immobilisierte thermoresponsive Polymere durch ein breites Temperaturprofil des Phasenübergangs aus. So wurde gefunden, dass für bestimmte Typen adhärenter Zellen eine Abkühlung von 37 °C auf Temperaturen unterhalb von 20 °C von bis zu einer Stunde erforderlich sind, um die Adhäsion von der Oberfläche des Substrats zu lösen (siehe Application Notes für die PNIPam-beschichteten UpCell-Kultivierungssubstrate der Fa. Nunc). Eine derartige Abkühlung über diesen Zeitraum ist jedoch wegen der damit verbundenen Beeinflussung der Zellfunktion unerwünscht. Des Weiteren wurde in der Praxis festgestellt, dass konventionelle thermoresponsive Polymerschichten für verschiedene Zelllinien, wie z.B. MCF7-Tumorzellen oder MG63 Osteoblasten-Zellen, unzureichend wirksam sind.

Herkömmliche Techniken zeichnen sich ferner durch Nachteile bei der Kultivierung mit so genannten Kokulturen aus. Da ein zu kultivierender Zelltyp zum Wachstum oder zur Aufrechterhaltung der Vitalität im adhärenten Zustand Botenstoffe (parakrine Faktoren) von anderen Zellen benötigt, müssen häufig die Kultivierung, das Wachstum oder manipulative oder analytische Prozesse von adhärenten Zellen in der Kokultur gemeinsam durchgeführt werden (z.B. Stammzellen und Feederzellen oder Melanozyten und Keratinozyten). Für die anschließende Trennung der Zellen stehen bisher nur Verfahren zur Verfügung, die auf einer Zelltrennung in flüssigen Zellsuspensionen basieren. Hierzu müssen die Zellen vom Substrat abgelöst und in eine Trennvorrichtung (Durchfluss-Zytometer) überführt werden, was wegen des Zeit- und Präparationsaufwandes und der geringen Ausbeute erhebliche Nachteile hat. Insbesondere für Proben mit Zellzahlen unterhalb von 10⁵ Zellen ist die herkömmliche Zelltrennung nicht durchführbar, da bei der Bildung der Suspension und der Trennung im Durchfluss-Zytometer übermäßig viele Zellen verloren gehen.

Die Aufgabe der Erfindung ist es, ein verbessertes thermoresponsives Substrat, insbesondere zur Aufnahme biologischer Zellen, bereitzustellen, mit dem Nachteile der herkömmlichen Technik überwunden werden. Die Aufgabe der Erfindung ist es insbesondere, ein verbessertes thermoresponsives Substrat bereitzustellen, das sich durch eine einfache Herstellung, eine hohe Flexibilität bei der Einstellung von Oberflächeneigenschaften, eine erweiterte Funktionalisierungsfähigkeit, eine Eignung für eine erweiterte Zahl von Zelltypen und/oder eine Eignung für eine schonende Zellkultivierung und Adhäsionssteuerung mit geringen Temperaturunterschieden auszeichnet. Eine weitere Aufgabe der Erfindung ist es, ein verbessertes Verfahren zur Herstellung eines thermoresponsiven Substrats, insbesondere zur Aufnahme biologischer Zellen, bereitzustellen, mit dem Nachteile herkömmlicher Verfahren zur Substratherstellung überwunden werden. Eine weitere Aufgabe der Erfindung ist es, ein verbessertes Kultivierungsverfahren unter Verwendung eines thermoresponsiven Substrats bereitzustellen, mit dem Nachteile und Beschränkungen herkömmlicher Kultivierungsverfahren überwunden werden.

Diese Aufgaben werden durch ein Substrat und Verfahren mit den Merkmalen der unabhängigen Ansprüche gelöst. Vorteilhafte Ausführungsformen und Anwendungen der Erfindung ergeben sich aus den abhängigen Ansprüchen.

Gemäß eines ersten Aspekts der vorliegenden Erfindung wird ein Substrat bereit gestellt, insbesondere zur Aufnahme biologischer Zellen, umfassend:
- einen Substratkörper, der eine Trägerfläche aufweist,
- eine oder mehrere Zwischenschichten Z, die auf der Trägerfläche aufgebracht ist/sind,
- eine auf der obersten Zwischenschicht angebrachte Schicht M, die Mikrogelpartikel enthält, welche zumindest ein thermoresponsives Polymer umfassen.

Gemäß eines zweiten Gesichtspunkts der Erfindung wird ein Verfahren zur Herstellung des erfindungsgemäßen Substrats bereitgestellt, bei dem thermoresponsive Polymerpartikel als Dispersion (Mikrogel, µ-Gel) hergestellt werden. Zur Bereitstellung der Schicht M, die die Mikrogelpartikel enthält, wird zunächst die Trägerfläche des Substratkörpers mit einer oder mehreren Zwischenschichten modifiziert und anschließend die Dispersion der Mikrogelpartikel auf die oberste Zwischenschicht aufgetragen. Dabei werden die Mikrogelpartikel, welche die oberste Zwischenschicht berühren, mit dieser verbunden, während überschüssige Mikrogelpartikel von der Trägerfläche getrennt, z.B. abgewaschen werden.

Gemäß eines dritten Gesichtspunkts der Erfindung wird ein Verfahren zur Kultivierung biologischer Zellen auf dem erfindungsgemäßen Substrat bereitgestellt, bei dem die biologischen Zellen auf dem Substrat in Kontakt mit den freiliegenden Mikrogelpartikeln angeordnet werden.

Das Verfahren zur Kultivierung biologischer Zellen auf dem erfindungsgemäßen Substrat umfasst folgende Schritte:
- Aufbringen von biologischen Zellen auf das Substrat,
- Einstellen von Kultivierungsbedingungen, so dass die biologischen Zellen einem Wachstum, einer Differenzierung und/oder einer Migration unterzogen werden.

Bevorzugt enthält zumindest eine Zwischenschicht des Substrats eine biologisch aktive Substanz oder biologische Zellen zur Erzeugung der biologisch aktiven Substanz, wobei das Substrat unter Bedingungen gehalten wird, so dass die biologisch aktive Substanz zu den biologischen Zellen auf dem Substrat diffundiert und ein Wachstum, eine Differenzierung und/oder eine Migration der Zellen auf dem Substrat bewirkt.

Die räumliche Trennung von Zielzellen, die sich auf der Oberfläche der Mikrogelpartikelschicht M befinden, und von Feeder-Zellen in einer der Zwischenschichten erleichtert die spätere spezifische Abtrennung der Zielzellen, ohne dass zusätzliche Maßnahmen zur Separierung der Zielzellen von den Feeder-Zellen notwendig sind.

Die erfindungsgemäße Bereitstellung eines Kultivierungssubstrats mit thermoresponsiven Mikrogelpartikeln hat eine Reihe von Vorteilen in Bezug auf die Einstellung physikalischer und/oder chemischer Oberflächeneigenschaften, die gezielte Veränderungen von Oberflächeneigenschaften, die Herstellung des Substrats und die Schaffung neuer Anwendungen oder Funktionen von Kultivierungssubstraten.

Der Phasenübergang zeichnet sich durch eine Änderung eines Festigkeitsparameters des thermoresponsiven Polymers (z. B. Härte, plastische oder elastische Deformierbarkeit, insbesondere Young'scher Elastizitätsmodul) aus. Mit dem Festigkeitsparameter ändert sich die Adhäsion von Zellen unter- und oberhalb einer kritischen Temperatur des Phasenübergangs. Gleichzeitig ändert sich der Wassergehalt des thermoresponsiven Polymers, wodurch die Adhäsion der Zellen ebenfalls beeinflusst wird. Die Einstellung der Adhäsion ist vorteilhafterweise mit größerer Zuverlässigkeit und Reproduzierbarkeit möglich als bei herkömmlichen Polymerschichten. Die Erfinder haben festgestellt, dass mit dem Phasenübergang der immobilisierten Polymerpartikel eine wesentlich erhöhte Anzahl von Oberflächenwechselwirkungen angeboten oder unterbrochen und damit die Zuverlässigkeit einer temperaturgesteuerten Freigabe von Zellen verbessert wird.

In Bezug auf die gezielte Veränderung von Oberflächeneigenschaften hat sich als besonders vorteilhaft erwiesen, dass erfindungsgemäße Substrate einer Funktionalisierung für die biologischen Zellen unterzogen werden können, ohne dass die thermoresponsiven Mikrogelpartikel ihr Ansprechverhalten verlieren. Vorteile für die Herstellung des erfindungsgemäßen Substrats ergeben sich aus der Stabilität der Partikeldispersion über Wochen oder Monate und der unmittelbaren Gebrauchsfertigkeit des Substrats nach der Beschichtung der Trägerfläche. Die Funktionalisierung thermoresponsiver Substrate liefert neue Anwendungen, z.B. für eine passive Steuerung einer Zellwanderung auf der Substratoberfläche.

Das erfindungsgemäße Substrat ist ein Kultivierungssubstrat für biologische Zellen. Das Substrat ist zur Aufnahme biologischer Zellen und zur Bereitstellung von physiologischen Kultivierungsbedingungen konfiguriert. Das Substrat ist insbesondere zur Aufnahme der Zellen in einem flüssigen Kultivierungsmedium eingerichtet, d.h. die Trägerfläche ist geeignet, das Kultivierungsmedium aufzunehmen. Der Substratkörper kann aus einem festen Material hergestellt sein, das starr oder nachgiebig (biegsam) ist. Das Material des Substratkörpers ist vorzugsweise temperaturstabil und insbesondere nicht thermoresponsiv. Die Trägerfläche ist vorzugsweise eine ebene Fläche, kann jedoch alternativ gekrümmt gebildet sein und andere, komplexe Geometrien aufweisen und Substrukturen umfassen.

Das thermoresponsive Polymer der Mikrogelpartikel weist bevorzugt in einem physiologischen Temperaturbereich einen Phasenübergang, insbesondere einen Volumenphasenübergang auf. Der Phasenübergang erfolgt vorzugsweise bei einer Temperatur unterhalb von 40 °C, insbesondere unterhalb von 37 °C, z.B. unterhalb von 35 °C. Bevorzugt erfolgt der Phasenübergang bei einer Temperatur oberhalb von 10 °C, insbesondere oberhalb von 20 °C, z.B. oberhalb von 30 °C. Das Temperaturintervall, in dem der Phasenübergang erfolgt, ist vorzugsweise schmaler als 15 °C, insbesondere schmaler als 10 °C, wie z.B. 5 °C oder weniger. Die Phasenübergangstemperatur wird über dynamische Lichtstreuung bestimmt.

In einer bevorzugten Ausführungsform ist das thermoresponsive Polymer ein neutrales Polymer.

Bevorzugt weist das thermoresponsive Polymer Monomereinheiten auf, die sich aus einem oder mehreren der folgenden Monomere ableiten:
- (Meth)Acrylsäureester,
- (Meth)Acrylsäureamid,
- einem vinylaromatischen Monomer,
- Vinylalkohol, der gegebenenfalls substituiert sein kann,
- Vinylether, der gegebenenfalls substituiert sein kann,
- Vinylamin, das gegebenenfalls substituiert sein kann,
- Vinylamid, das gegebenenfalls substituiert sein kann,
- Ethylenglycol, der gegebenenfalls substituiert sein kann,
- Propylenglycol, der gegebenenfalls substituiert sein kann,
- Ethylenimin, das gegebenenfalls substituiert sein kann,
- Oxazolin, das gegebenenfalls substituiert sein kann,
- C₅- und/oder C₆-Monosaccharide, wie z.B. D-Glucose, D-Mannose, D-Galactose, die gegebenenfalls substituiert sein können.

Bevorzugt wird das thermoresponsive Polymer aus einem der folgenden Polymere oder deren Copolymeren oder Gemischen ausgewählt:
(1) 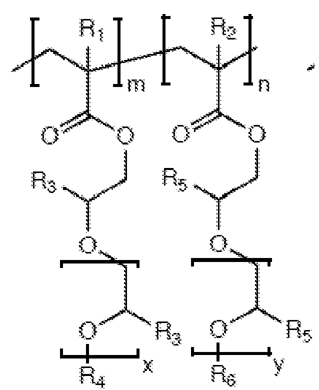 wobei
   R₁, R₂ = H, Alkyl wie z.B. C₁₋₄-Alkyl,
   R₃, R₅ = H, Alkyl wie z.B. C₁₋₄-Alkyl,
   R₄, R₆ = H, Alkyl wie z.B. C₁₋₄-Alkyl, Allyl, Phenyl, Benzyl, Acyl,
   x und y unabhängig voneinander 1-50, bevorzugter 2-20 sind,
   4≤ m + n ≤ 10⁷;
(2) 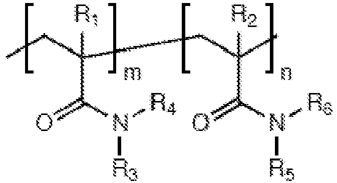 wobei
   R₁, R₂ = H, Alkyl wie z.B. C₁₋₄-Alkyl,
   R₃, R₄, R₅, R₆ = H, Alkyl wie z.B. C₁₋₄-Alkyl, Allyl, Phenyl, Benzyl,
   4≤ m + n ≤ 10⁷;
(3) 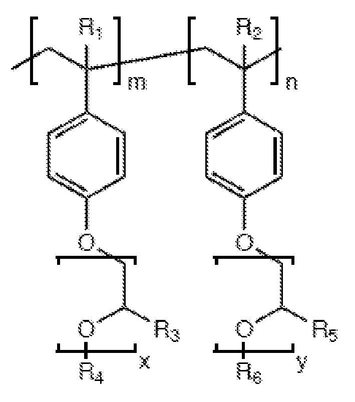 wobei
   R₁, R₂ = H, Alkyl wie z.B. C₁₋₄-Alkyl,
   R₃, R₅ = H, Alkyl wie z.B. C₁₋₄-Alkyl,
   R₄, R₆ = H, Alkyl wie z.B. C₁₋₄-Alkyl, Allyl, Phenyl, Benzyl, Acyl,
   x und y unabhängig voneinander 1-50, bevorzugter 2-20 sind,
   4≤ m + n ≤ 10⁷;
(4) 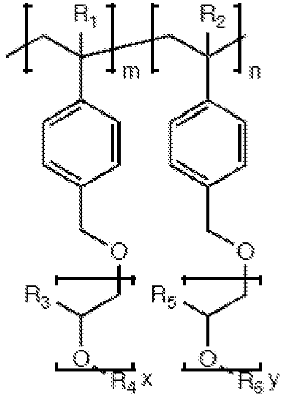 wobei
   R₁, R₂ = H, Alkyl wie z.B. C₁₋₄-Alkyl,
   R₃, R₅ = H, Alkyl wie z.B. C₁₋₄-Alkyl,
   R₄, R₆ = H, Alkyl wie z.B. C₁₋₄-Alkyl, Allyl, Phenyl, Benzyl, Acyl,
   x und y unabhängig voneinander 1-50, bevorzugter 2-20 sind,
   4≤ m + n ≤ 10⁷;
(5) 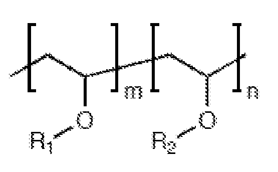 wobei
   R₁, R₂ = H, Alkyl wie z.B. C₁₋₄-Alkyl,
   4≤ m + n ≤ 10⁷;
(6) 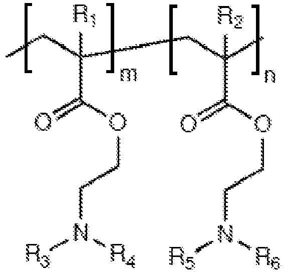 wobei
   R₁, R₂ = H, Alkyl wie z.B. C₁₋₄-Alkyl,
   R₃, R₅ = H, Alkyl wie z.B. C₁₋₄-Alkyl,
   R₄, R₆ = H, Alkyl wie z.B. C₁₋₄-Alkyl, Allyl, Phenyl, Benzyl, Acyl,
   4≤ m + n ≤ 10⁷;
(7) 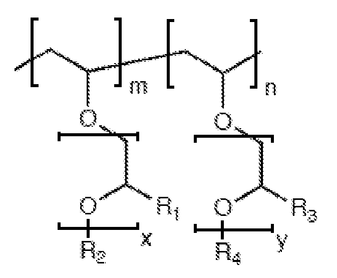 wobei
   R₁, R₃ = H, Alkyl wie z.B. C₁₋₄-Alkyl,
   R₂, R₄ = H, Alkyl wie z.B. C₁₋₄-Alkyl, Allyl, Phenyl, Benzyl, Acyl,
   x und y unabhängig voneinander 1-50, bevorzugter 2-20 sind,
   4≤ m + n ≤ 10⁷;
(8) 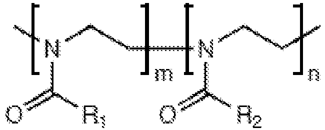 wobei
   R₁, R₂ = H, Alkyl wie z.B. C₁₋₄-Alkyl, Allyl, Phenyl, Benzyl,
   4≤ m + n ≤ 10⁷;
(9) 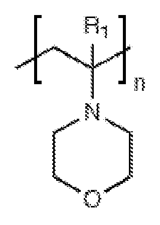 wobei
   R₁, = H, Alkyl wie z.B. C₁₋₄-Alkyl,
   n > 5;
(10) 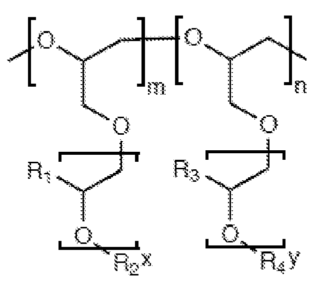 wobei
   R₁, R₃ = H, Alkyl wie z.B. C₁₋₄-Alkyl,
   R₂, R₄ = H, Alkyl wie z.B. C₁₋₄-Alkyl, Allyl, Phenyl, Benzyl, Acyl,
   x und y unabhängig voneinander 1-50, bevorzugter 2-20 sind,
   4≤ m + n ≤ 10⁷;
(11) 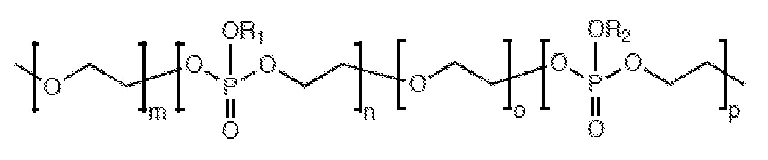 wobei
   R₁, R₂ = H, Alkyl wie z.B. C₁₋₄-Alkyl,
   4≤ m + n + o + p ≤ 10⁷;
(12) 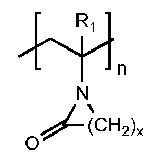 wobei
   R₁ = H, Alkyl wie z.B. C₁₋₄-Alkyl,
   x 2 bis 6 ist,
   n größer 5 ist, z.B. 6 ≤ n ≤ 10⁷
(13) 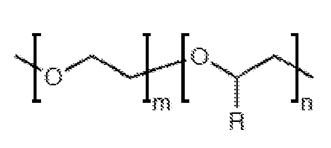 wobei
   R = Alkyl, bevorzugt Methyl,
   10 > m : n > 0,1;
   4≤ m + n ≤ 10⁷;
(14) 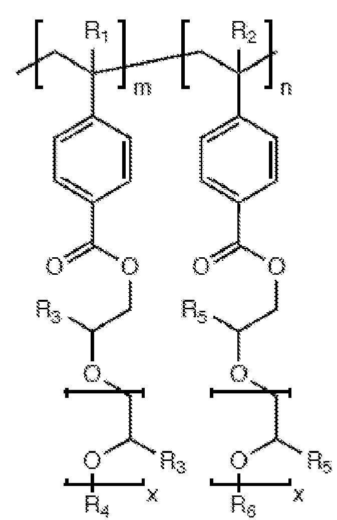 wobei
   R₁, R₂ = H, Alkyl wie z.B. C₁₋₄-Alkyl,
   R₃, R₅ = H, Alkyl wie z.B. C₁₋₄-Alkyl,
   R₄, R₆ = H, Alkyl wie z.B. C₁₋₄-Alkyl, Allyl, Phenyl, Benzyl, Acyl,
   x und y unabhängig voneinander 1-50, bevorzugter 2-20 sind,
   4≤ m + n ≤ 10⁷;
(15) 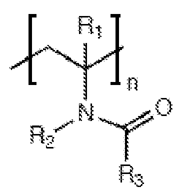 wobei
   R₁, R₂ = H, Alkyl wie z.B. C₁₋₄-Alkyl,
   R₃ = Alkyl wie z.B. C₁₋₄-Alkyl, Allyl, Phenyl, Benzyl,
   4≤ n ≤ 10⁷;
(16) 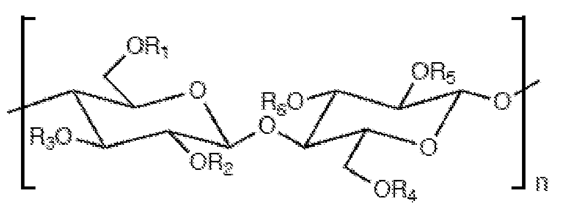 wobei
   R₁, R₂, R₃, R₄, R₅, R₆ = H, Alkyl wie z.B. C₁₋₄-Alkyl, Allyl, Phenyl, Benzyl, Acyl,
   4≤ n ≤ 10⁷.

Die Trägerfläche kann die unmittelbare Oberfläche des Substratkörpers (z. B. aus Glas, Siliziumwafer oder Kunststoffe wie Polystyrol, COP, Polycarbonat) sein oder durch einen Metallfilm, z.B. aus Gold, Silber, Platin, Titan oder Chrom, auf dieser gebildet werden.

Die Mikrogelpartikel auf der obersten Zwischenschicht können sämtlich identisch aus einem einzigen Polymer gebildet sein. Wenn gemäß einer alternativen Variante die Mikrogelpartikel aus mindestens zwei verschiedenen Polymeren gebildet sind, können sich Vorteile bei der Einstellung der Oberflächeneigenschaften ergeben. Des Weiteren können in diesem Fall Mikrogelpartikel mit verschiedenen Zusammensetzungen auf der obersten Zwischenschicht angeordnet sein. Mikrogelpartikel mit verschiedenen Zusammensetzungen können z.B. nach Teilbereichen getrennt vorgesehen sein, um verschiedene Kultivierungsbedingungen bereitzustellen. Alternativ können die Mikrogelpartikel mit den verschiedenen Zusammensetzungen miteinander vermischt auf der Oberfläche verteilt angeordnet sein. Alternativ oder zusätzlich können die Mikrogelpartikel verschiedene Durchmesser aufweisen. Beispielsweise können Mikrogelpartikel mit verschiedenen Durchmessern getrennt in verschiedenen Teilbereichen der Oberfläche fixiert oder miteinander vermischt über die Oberfläche verteilt angeordnet sein.

Vorteilhafterweise können Mikrogele mit einem vorbestimmten Durchmesser der dispergierten, kolloidalen Teilchen hergestellt werden (siehe M. Andersson, S.L. Maunu, J. Polym. Sci. B 44 (2006) 3305; X. Wu et al. Coll. Poly. Sci. 272 (1994) 467). Dies ermöglicht, die Größe, oder, falls Partikel mit verschiedenen Durchmessern angeordnet werden, die verschiedenen Größen der thermoresponsiven Mikrogelpartikel gezielt einzustellen. Gemäß bevorzugten Ausführungsformen der Erfindung haben die thermoresponsiven Mikrogelpartikel einen Durchmesser von mindestens 10 nm, insbesondere mindestens 20 nm, besonders bevorzugt mindestens 50 nm, wie z.B. mindestens 100 nm. Die Obergrenze des Partikeldurchmessers beträgt vorzugsweise 50 µm. Besonders bevorzugt ist der Durchmesser der thermoresponsiven Mikrogelpartikel kleiner oder gleich 30 µm, insbesondere kleiner oder gleich 20 µm, wie z.B. 10 µm oder kleiner, z.B. kleiner als 1 µm.

Gemäß einer weiteren Variante können die thermoresponsiven Mikrogelpartikel eine Kern-Schale-Struktur aufweisen, wobei ein Kern entweder aus einem nichtthermoresponsiven Material, insbesondere einem festen Trägerpartikel oder aus einem vernetzten thermoresponsiven Material bestehen kann. Die Schale besteht aus einem thermoresponsiven Polymermaterial. Die Verwendung des festen Trägerpartikels, der z.B. aus anorganischem Glas, Metall, Keramik oder Kunststoff, insbesondere Polymethylmethacrylat oder Polystyrol, gebildet sein kann, kann Vorteile in Bezug auf die Bereitstellung einer bestimmten Mindesthärte der Oberfläche des erfindungsgemäßen Substrats haben. Weiterhin bieten Kern-Schale-Partikel die Möglichkeit, unvernetzte oder schwach vernetzte Polymerketten in die Hülle des Polymerpartikels zu integrieren, ohne den mechanischen Zusammenhalt des Partikels zu beeinträchtigen. Unvernetzte oder schwach vernetzte Hüllen der Polymerpartikel bieten den Vorteil, daß die thermoresponsiven Ketten sehr beweglich bleiben und so die Konformationsänderung beim Phasenübergang besser zum Tragen kommen kann. Sofern schwach vernetzte Partikel zum Einsatz kommen, soll die Vernetzungsdichte nicht mehr als 1 pro 20 Wiederholungseinheiten betragen und bevorzugt zwischen 1 pro 100 und 1 pro 500 Wiederholungseinheiten liegen. Um einen ausgeprägten thermoresponsiven Effekt zu erreichen, soll die Dicke der Schale mindestens 10 nm und höchstens 400 nm, bevorzugt zwischen 30 und 100 nm betragen. Generelle Beispiele für die Herstellung von Kern-Schale-Partikeln finden sich zum Beispiel in Kolloid Z. Z. Polym. 211, 113-121 (1966), Progr. Colloid Polym. Sci. 101, 178-183 (1996), oder Macromolecules 39, 3154-3160 (2006).

Vorzugsweise ist die Dicke der Schicht aus thermoresponsiven Polymerpartikeln auf der Trägerfläche kleiner oder gleich dem Durchmesser der Polymerpartikel.

Bevorzugt liegen die Mikrogelpartikel der Schicht M in Form einer Monolage, bevorzugt einer geschlossenen Monolage vor.

Alternativ zu einer geschlossene Monolage der Mikrogelpartikel in der Schicht M kann im Rahmen der vorliegenden Erfindung auch eine Sub-Monolage mit Lücken zwischen den Mikrogelpartikeln vorgesehen sein.

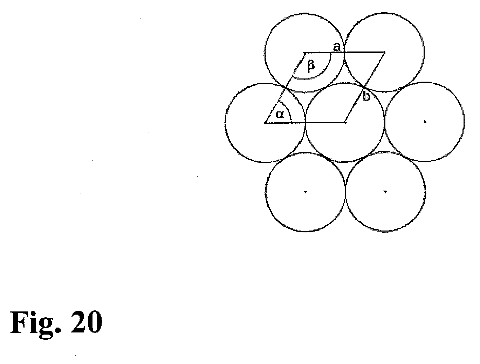

In einer bevorzugten Ausführungsform liegen die Mikrogelpartikel in der Monolage in einer geordneten Struktur vor. Bevorzugt weist die Monolage eine Packungsdichte auf, die im Bereich von einer dichtesten Kugelpackung bis zu 0,15, bevorzugter 0,25 Mikrogelpartikel pro zweidimensionaler hexagonaler Elementarzelle liegt. Unter einer zweidimensionalen hexagonalen Elementarzelle (d.h. eine zweidimensionale Elementarzelle mit hexagonaler Symmetrie) wird ein Parallelogramm mit a=b, α=γ=60°, β=δ=120° verstanden. Eine zweidimensionale hexagonale Elementarzelle ist schematisch in Fig. 20 dargestellt.

Bevorzugt liegt auf der die Mikrogelpartikel enthaltenden Schicht M biologisches Material, z.B. biologische Zellen, Proteine, Proteinaggregate, Mikroorganismen, oder Kombinationen davon, vor.

Wie oben dargelegt, enthält das erfindungsgemäße Substrat eine oder mehrere Zwischenschichten, die zwischen dem Substratkörper und der die Mikrogelpartikel enthaltenden Schicht M angeordnet ist/sind.

Die beschriebenen Multischichtsysteme bieten spezielle Vorteile gegenüber Monolagen. So zeichnen sich bei polymerbasierten Systemen Multischichten durch eine größere Robustheit gegenüber Defekten aus. Außerdem zeichnen sich Multischichten gegenüber Monoschichten durch eine erhöhte Rauigkeit aus, was durch die erhöhte Bindungsfläche die Immobilisierung der µ-Gele auf dem Träger verbessert.

Solche Multischichten können beispielsweise ausschließlich aus Mikrogelpartikeln oder aus Mikrogelpartikeln und anderen Komponenten (niedermolekulare homo- oder heterobi- oder multifunktionale Verbindungen, organische Polymere ohne oder mit thermoresponsivem Verhalten, anorganische Polymere oder Partikel) aufgebaut sein. Abfolgen der Schichten können alternierend, komplex regelmäßig oder auch regellos sein.

Die Dicke einer Zwischenschicht kann über einen breiten Bereich variiert werden. Beispielsweise kann die Dicke einer Zwischenschicht im Bereich von 0,2 nm bis 50 µm oder 2 nm bis 30 µm oder auch 5 nm bis 10 µm liegen.

Hinsichtlich bevorzugter nicht-partikulärer, isotroper bzw. homogener Polymerschichten als Zwischenschicht können beispielsweise Folgende genannt werden:
- Alternierende Schichten aus Polymeren
   mit Aktivester- bzw. Aminogruppen,
   oder mit Epoxy- bzw. Aminogruppen,
   oder mit Thiol- bzw. Maleiimidgruppen,
   oder mit Vinylsulfon- bzw. Thiolgruppen,
   oder mit Alkin- bzw. Azidgruppen;
- Schichten aus Polymeren mit vernetzbaren Gruppen, insbesondere photovernetzbaren Gruppen.

Hinsichtlich geeigneter poröser Materialien können beispielhaft poröse Sol-Gel-Schichten (z.B. aus Metallalkoxyden), Nanokompositfilme (wie z.B. beschrieben in Brinker et al., Progress in Organic Coatings 47 (2003) 393-400) Zeolithe, poröse Stärke- oder Cellulose-Kugeln oder auch poröse Membranen oder Kernspurfilter genannt werden.

Wie nachfolgend noch diskutiert wird, kann in einer bevorzugten Ausführungsform zumindest eine der Zwischenschichten eine aktive Substanz, insbesondere eine biologisch aktive Substanz wie parakrine Faktoren, Hormone und/oder Enzyme bereitstellen. Damit diese aktiven Substanzen die Zwischenschicht(en) problemlos passieren und so zu den Zielzellen, die auf der Mikrogelpartikelschicht M vorliegen können, gelangen, kann es vorteilhaft sein, wenn in der Zwischenschichten bzw. den Zwischenschichten poröse Materialien vorliegen.

Liegen in zumindest einer Zwischenschicht Zellen vor, so handelt es sich bevorzugt um Zellen, die zur Abgabe biologisch aktiver Substanzen fähig sind und diese biologisch aktiven Substanzen eine spezifische Wechselwirkung mit den auf der Mikrogelpartikelschicht vorliegenden biologischen Zellen eingehen können. Die biologisch aktiven Substanzen können z.B. spezifische zelluläre Reaktionen bei den Zielzellen auslösen.

Substanzen, welche zelluläre Reaktionen auslösen, sind allgemein Substanzen, welche durch Bindung an Oberflächenrezeptoren der biologischen Zellen z.B. eine verstärkte Adhäsion, eine Migration (Zellwanderung), eine Differenzierung (insbesondere Stammzelldifferenzierung), eine Änderung des Aktivierungsstatus oder eine Änderung der Malignität bewirken.

Einen besonderen Vorteil von Multischichtsystemen stellt die Möglichkeit einer vertikal separierten Anordnung funktionaler Elemente dar. Z. B. können Schichten in den Aufbau integriert werden, die die Möglichkeit der zeitlich verzögerten oder kontrollierten Freisetzung von biologisch aktiven Substanzen bieten. Auch der Einbau von Zellen in Multischichtsysteme ist möglich. Dies kann zum Beispiel genutzt werden, um Zellen, die parakrine Faktoren absondern, in einer der Schichten unterhalb der äußeren thermoresponsiven Mikrogelschicht zu immobilisieren. Dadurch vermeidet man eine Mischung von Feeder-Zellen mit der Zielspezies, so dass diese nach Abschluss der Kultivierung ohne zusätzlichen Aufwand isoliert werden kann.

Bevorzugt wird die biologisch aktive Substanz ausgewählt aus parakrinen Faktoren, Hormonen, Enzymen, biochemischen Transmittern oder Gemischen bzw. Kombinationen davon.

Im Rahmen der vorliegenden Erfindung ist es möglich, dass die biologisch aktive Substanz in der Zwischenschicht in Mikrogelpartikeln, Mikrokapseln, Vesikeln, Liposomen oder einem porösen Material oder Kombinationen davon vorliegt. Alternativ kann die biologisch aktive Substanz durch ein geeignetes, in der Zwischenschicht vorliegendes biologisches Material, insbesondere eine biologische Zelle erzeugt werden, um dann durch die Zwischenschicht in die die Mikrogelpartikel enthaltende Schicht M zu diffundieren und mit Zielzellen, die auf dieser Schicht M angebracht sind, in Wechselwirkung zu treten.

In einer bevorzugten Ausführungsform weist das Substrat mindestens zwei Zwischenschichten Z1 und Z2 auf, wobei die Zwischenschicht Z1 biologische Zellen, die bevorzugt biologisch aktive Substanzen wie z.B. parakrine Faktoren absondern können, enthält, und die Zwischenschicht Z2 aus einem zellkompatiblen Material besteht.

Bevorzugt ist die Zwischenschicht Z2 unmittelbar auf der Zwischenschicht Z1 angebracht, d.h. die Zwischenschichten Z1 und Z2 sind benachbart. Die Schicht M, die die thermoresponsiven Mikrogelpartikel umfasst, kann unmittelbar auf der Zwischenschicht Z2 aufgebracht sein. Alternativ können zwischen der Zwischenschicht Z2 und der Mikrogelpartikelschicht M noch eine oder mehrere zusätzliche Zwischenschichten angebracht sein.

Bevorzugt wird das zellkompatible Material der zweiten Schicht so ausgewählt, dass die von den biologischen Zellen abgesonderten Materialien problemlos durch diese Zwischenschicht Z2 hindurch diffundieren können.

Geeignete zellkompatible Zwischenschichten, die gegebenenfalls auch porös sein können, umfassen zum Beispiel Polysaccharide, Poly(vinylpyrrolidon), Poly(diallyldimethylammoniumchlorid), Polylysin, Polyhyaluronsäure, oder deren Gemische.

Als Materialien für zellkompatible Zwischenschichten sind auch Polyelektrolyten geeignet, mit denen Layer-by-Layer-Schichtsysteme aufgebaut werden können, insbesondere biokompatible Polyelektrolyten wie Poly-Lysin und Hyaluronsäure.

Bevorzugt handelt es sich bei den Mikrokapseln, Vesikeln und/oder Liposomen um responsive Mikrokapseln, Vesikeln und/oder Liposomen. Im Rahmen der vorliegenden Erfindung werden unter responsiven Mikrokapseln, Vesikeln und/oder Liposomen solche verstanden, die unter Einfluss einer äußeren Einwirkung, insbesondere unter Einwirkung von Licht oder durch Änderung der Ionenstärke, in der Lage sind, in ihnen gespeicherte Substanzen frei zu setzen. Solche Mikrokapseln, Vesikeln und/oder Liposomen sind dem Fachmann grundsätzlich bekannt.

Der Aufbau von Multischichtsysteme kann mit Hilfe verschiedener Techniken, wie z. B. Adsorption aus Lösung (), Chemical Self Assembly (D. Elbert et al., Biomacromolecules 8, 3682-3686 (2007)), Spin Coating (P. A. Chiarelli et al., Langmuir 18, 168-173 (2002)), Dip Coating, Solution Casting (S. Cheng et al., Polymer 50, 2775-2785 (2009)), Plasmapolymerisation (J. Timmons et al., Journal of Biomedical Materials Research 36, 181-189 (1997)) oder Chemical Vapour Deposition (K. K. Gleason et al., Advanced Functional Materials 19, 1276-1286 (2009)) erfolgen, der Zusammenhalt der Schichten kann durch elektrostatische Wechselwirkungen (G. Decher, J.-D. Hong, Die Makromolekulare Chemie Macromolecular Symposia 46, 312-327 (1991)), Komplexbildung (B. Tieke et al., Langmuir 17, 7706-7709 (2001)), Nebenvalenzwechselwirkungen wie z. B. Wasserstoffbrückenbindungen (Rubner et al., Macromolecules 30, 2717-2725 (1997)) oder durch kovalente Bindungen (Kunitake et al., Chemistry Letters 1996, 831-832) gewährleistet sein. Des Weiteren können spezifische biologische Rezeptor-Ligand-Bindungen, wie z.B. die Bindung von Streptavidin und Biotin (G. Decher et al., Supramolecular Science 5, 309-315 (1998)) genutzt werden, indem z. B. biotinylierte Polymere an Streptavidinschichten gebunden werden. Auch Kombinationen der genannten Verfahren und Aufbauprinzipien sind möglich und u. U. besonders wünschenswert, um unterschiedlichen Schichten ihrer Aufgabe besonders angepaßte Materialeigenschaften zu verleihen. Auch die Fixierung der terminalen thermoresponsiven Mikrogelschicht kann auf eine der oben genannten Weisen gewährleistet werden.

In einer bevorzugten Ausführungsform sind die Mikrogelpartikel der Schicht M über kovalente Bindungen mit der benachbarten Zwischenschicht verknüpft.

Gemäß einer weiteren bevorzugten Ausführungsform der Erfindung ist das Substrat mit mindestens einer Modulatorsubstanz ausgestattet.

Die Modulatorsubstanz kann in einer Zwischenschicht und/oder der die Mikrogelpartikel enthaltenden Schicht M immobilisiert vorliegen.

Die mindestens eine Modulatorsubstanz kann zwischen den Mikrogelpartikeln der Schicht M angeordnet sein.

Die Bereitstellung der mindestens einen Modulatorsubstanz ermöglicht vorteilhafterweise eine Funktionalisierung des Substrats und eine Beeinflussung der Kultivierungsbedingungen auf dem Substrat, ohne dass das Temperaturverhalten der Mikrogelpartikel beeinträchtigt wird.

Vorteilhafterweise können verschiedene Typen von Modulatorsubstanzen einzeln oder in Kombination vorgesehen sein. Beispielsweise können Modulatorsubstanzen vorgesehen sein, welche die Adhäsionsfähigkeit der biologischen Zellen steigern (adhäsionssteigernde Modulatorsubstanzen, zellanziehende Moleküle). Hierzu ist mindestens eine der folgenden Substanzen vorgesehen:
- Biomoleküle, wie z.B. Fibronektin, Kollagen, Laminin,
- adhäsionsvermittelnde Peptide, wie z.B. Peptide, welche die Aminosäurefolge RGD enthalten,
- synthetische Polymere, wie z.B. Poly-L-Lysin, Polystyrolsulfonat, Polyallylamin, Polyethylenimin.

Alternativ können Modulatorsubstanzen vorgesehen sein, welche die Adhäsionsfähigkeit der biologischen Zellen vermindern (adhäsionsmindernde Modulatorsubstanzen, zellabstoßende Molekülen). In diesem Fall wird mindestens eine der folgenden Substanzen als Modulatorsubstanz verwendet:
- Proteine, wie z.B. Rinderserumalbumin (bovine serum albumin, BSA),
- adhäsionsmindernde Peptide, wie z.B. Peptide mit hohem Anteil an Leucin und Isoleucin,
- synthetische Polymere, wie z.B. Polymere, die Ketten aus Polyethylenglycol ("PEG") enthalten, und
- Lipide.

Es können des Weiteren adhäsionssteigernde und adhäsionsmindernde Modulatorsubstanzen auf einem Substrat kombiniert vorgesehen sein. Beispielsweise können die Modulatorsubstanzen mit verschiedenen Wirkungen in verschiedenen Teilbereichen der Trägerfläche bzw. in verschiedenen Zwischenschichten getrennt oder miteinander vermischt angeordnet sein. In letzterem Fall kann durch das Mischungsverhältnis von adhäsionssteigernden und adhäsionsmindernden Modulatorsubstanzen eine effektive Adhäsionsfähigkeit in der Oberfläche des Substrats bzw. der zur Schicht M benachbarten Zwischenschicht eingestellt werden.

Gemäß einer weiteren Variante der Erfindung kann alternativ oder zusätzlich eine Modulatorsubstanz vorgesehen sein, die geeignet ist, in den biologischen Zellen zelluläre Reaktionen zu induzieren. Beispielsweise kann vorgesehen sein, dass eine Modulatorsubstanz an Oberflächenrezeptoren der Zellen anbindet, um spezifische zelluläre Reaktionen auszulösen. Für diese Funktion sind Substanzen, wie z.B. Proteine der extrazellulären Matrix (ECM) wie etwa Fibronektin, Antikörper gegen Rezeptoren (EGFR), die Wachstumsfaktoren binden, oder Antikörper z.B. gegen CD 28 und CD 3 von T-Zellen (Aktivierung der Immunantwort) besonders bevorzugt vorgesehen.

Ein weiterer Vorteil der Erfindung ergibt sich aus der Flexibilität der Bereitstellung der mindestens einen Modulatorsubstanz. Gemäß einer ersten Variante sind Modulatorpartikel vorgesehen, die aus der mindestens einen Modulatorsubstanz bestehen oder mit dieser beschichtet sind und zwischen den Mikrogelpartikeln in der Schicht M und/oder in einer Zwischenschicht angeordnet sind. Vorteilhafterweise können die Modulatorpartikel dem Mikrogel zugesetzt und mit diesem auf die oberste Zwischenschicht aufgetragen werden. Alternativ oder zusätzlich kann die mindestens eine Modulatorsubstanz als Substanzschicht auf der Trägerfläche gebildet sein, auf der die Mikrogelpartikel der Schicht M angeordnet sein können.

Vorteilhafterweise kann gemäß einer weiteren Ausführungsform der Erfindung eine räumliche Modulation der Oberflächeneigenschaften des Substrats auf der Trägerfläche vorgesehen sein. Die Trägerfläche weist in mindestens zwei Teilbereichen verschiedene Oberflächeneigenschaften auf. Vorteilhafterweise können die Teilbereiche dadurch gebildet werden, dass mindestens eines von den Mikrogelpartikeln der Schicht M, einer Zwischenschicht und der mindestens einen Modulatorsubstanz auf der Trägerfläche mit mindestens einem räumlichen Dichtegradienten angeordnet wird. Mindestens eine der genannten Komponenten der Oberfläche des Substrats ist mit einer räumlichen Dichte vorgesehen, die in mindestens einer Richtung entlang der Trägerfläche veränderlich ist. Der Dichtegradient kann stufenweise oder kontinuierlich gebildet sein. Die Bereitstellung des mindestens einen Dichtegradienten ermöglicht vorteilhafterweise, dass die Zellen entlang der Trägerfläche verschiedene Adhäsionsfähigkeiten, verschiedene Temperaturreaktionen, verschiedene zelluläre Reaktionen, wie z.B. verschiedene Differenzierungen, und/oder verschiedene Zellwanderungen zeigen.

Gemäß einer weiteren vorteilhaften Ausführungsform der Erfindung kann das Substrat mindestens eine Kultivierungskavität aufweisen. Die Kultivierungskavität ist ein über die Substratoberfläche hinausragender Vorsprung, der die Substratoberfläche partiell überdeckend ausgebildet ist. Die Kultivierungskavität umfasst z.B. die Gestalt eines einseitig offenen Hohlraums oder einer Tasche, und sie ist zur Aufnahme von mindestens einer biologischen Zelle eingerichtet. Mit der Kultivierungskavität werden räumliche Kultivierungsbedingungen nachgebildet, die bei der Kultivierung im Zellverband gegeben sind.

Vorteilhafterweise kann der mindestens eine Dichtegradient zur Funktionalisierung der Oberfläche des Substrats so gebildet sein, dass Zellen zu der Kultivierungskavität wandern, um dort einer weiteren Kultivierung und/oder Differenzierung unterzogen zu werden.

Das erfindungsgemäße Substrat ist für die Kultivierung biologischer Zellen ausgelegt. Hierzu ist der Substratkörper vorzugsweise ein Teil einer Kultivierungseinrichtung, wie z.B. eines Kultivierungsgefäßes oder einer Fluidikeinrichtung, in der Zellen kultivierbar sind, wie z.B. eines fluidischen Mikrosystems. Der Substratkörper kann mit der Kultivierungseinrichtung fest verbunden sein, z.B. den Boden des Kultivierungsgefäßes bilden, oder von der Kultivierungseinrichtung lösbar sein, z.B. ein in ein Kultivierungsgefäß einlegbares Teil darstellen.

Das erfindungsgemäße Verfahren zur Kultivierung biologischer Zellen kann mit einem oder mehreren der folgenden Verfahrensschritte ausgeführt werden. So kann eine Einstellung der Adhäsion der biologischen Zellen auf dem Substrat vorgesehen sein, indem die Temperatur des Substrats eingestellt wird. Die Temperatureinstellung kann global für das gesamte Substrat oder lokal für mindestens einen Teilbereich vorgesehen sein. Mit der Temperatureinstellung wird ein Festigkeitsparameter der Substratoberfläche beeinflusst. Des Weiteren kann eine Einstellung einer zelltypspezifischen Migration von mindestens einem Typ der biologischer Zellen vorgesehen sein, indem mindestens ein Zelltyp, z. B. mindestens ein Differenzierungstyp, entlang eines Dichtegradienten einer Modulatorsubstanz zur Migration angeregt wird. Des Weiteren kann eine Einstellung der Migration von mindestens einem Zelltyp mittels des Dichtegradienten der Modulatorsubstanz derart vorgesehen sein, dass die biologischen Zellen in eine Kultivierungskavität wandern.

Weitere Einzelheiten und Vorteile der Erfindung werden im Folgenden unter Bezug auf die beigefügten Zeichnungen erläutert. Es zeigen:
- Figur 1:: eine schematische Perspektivansicht einer ersten Ausführungsform des erfindungsgemäßen Substrats;
- Figur 2:: schematische Illustrationen des Phasenübergangs von thermoresponsiven Polymerpartikeln;
- Figur 3:: experimentelle Ergebnisse, die den Phasenübergang von thermoresponsiven Polymerpartikeln zeigen;
- Figur 4:: eine schematische Illustration eines thermoresponsiven Polymerpartikels mit Kern-Schale-Struktur;
- Figuren 5 bis 8:: weitere Ausführungsformen erfindungsgemäßer Substrate;
- Figur 9:: eine schematische Illustration einer Kultivierungseinrichtung, die mit dem erfindungsgemäßen Substrat ausgestattet ist;
- Figuren 10 bis 13:: schematische Illustrationen weiterer Ausführungsformen erfindungsgemäßer Substrate, die mit mindestens einer Modulatorsubstanz ausgestattet sind;
- Figuren 14 bis 17:: schematische Illustrationen weiterer Ausführungsformen erfindungsgemäßer Substrate mit Dichtegradienten von Oberflächenkomponenten; und
- Figur 18:: eine schematische Illustration eines erfindungsgemäßen Substrats mit einer Kultivierungskavität;
- Figur 19:: eine schematische Illustration eines erfindungsgemäßen Substrats, das eine Zwischenschicht mit Feeder-Zellen aufweist,
- Figur 20:: eine schematische Darstellung einer zweidimensionalen hexagonalen Elementarzelle.

Bevorzugte Ausführungsformen der Erfindung werden im Folgenden unter Bezug auf die Bereitstellung von einer oder mehreren Zwischenschichten und einer darauf angeordneten Schicht M mit Mikrogelpartikeln, die ein thermoresponsives Polymer enthalten, und die optional vorgesehene Funktionalisierung beschrieben. Einzelheiten von Kultivierungsverfahren, insbesondere von Verfahren zur Handhabung biologischer Zellen und deren gezielte Beeinflussung, werden nicht beschrieben, da diese an sich aus dem Stand der Technik bekannt sind. Des Weiteren wird betont, dass die beigefügten Zeichnungen schematische, vergrößerte Illustrationen von Ausschnitten des erfindungsgemäßen Kultivierungssubstrats darstellen. Die Umsetzung der Erfindung in der Praxis ist nicht auf die Illustrationen beschränkt, sondern mit abgewandelten Formen, Größen und Zusammensetzungen des Substrats möglich.

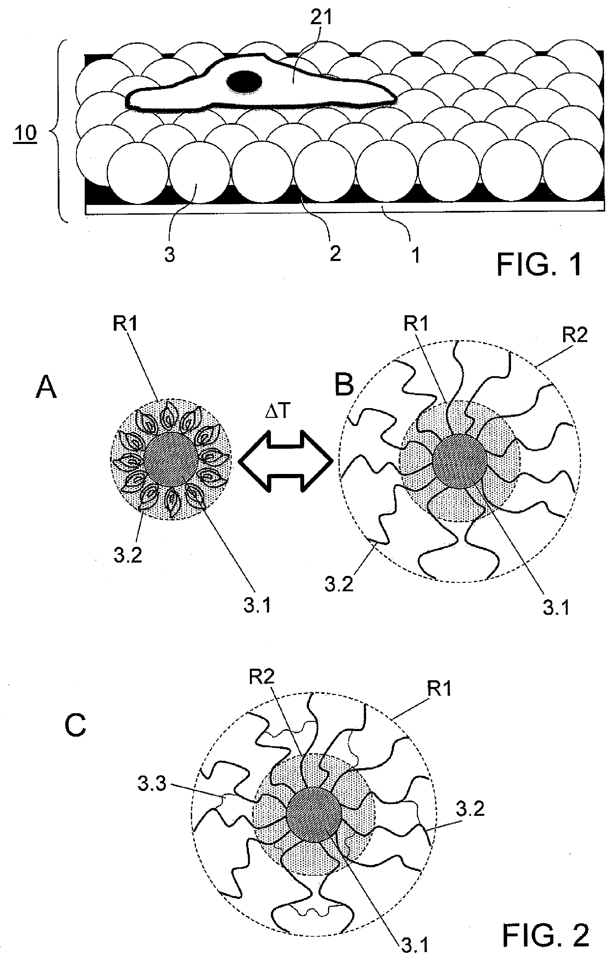

Figur 1 zeigt in schematischer Perspektivansicht eine erste Ausführungsform des erfindungsgemäßen Substrats 10 mit einer Zwischenschicht 1, auf deren Oberseite 2 Mikrogelpartikel 3, welche ein thermoresponsives Polymer enthalten, angeordnet sind. Die Monolage dieser Mikrogelpartikel 3 bildet die Schicht M. Die Zwischenschicht ist auf einem Substratkörper angebracht, der jedoch in Figur 1 nicht gezeigt wird. Der Substratkörper 1 besteht z. B. aus Metallen wie Gold, Titan, Platin, Glas, Siliziumwafer oder Kunststoffe wie Polystyrol, COP, Polycarbonat, dessen Oberfläche die Trägerfläche 2 bildet. Die thermoresponsiven Mikrogelpartikel 3 sind aus den kolloidalen Bestandteilen eines Mikrogels gebildet. Abweichend von der schematisch illustrierten Kugelform können die thermoresponsiven Mikrogelpartikel 3 in der Praxis z.B. eine Halbkugelform oder eine in Abhängigkeit von den Beschichtungsbedingungen unregelmäßig deformierte Gestalt aufweisen.

Die thermoresponsiven Mikrogelpartikel 3, die z.B. aus PNIPam hergestellt sind, zeigen den in den Figuren 2A bis 2C schematisch illustrierten Phasenübergang. Die thermoresponsiven Mikrogelpartikel 3 umfassen einen vernetzten Kern 3.1, von dem radial nach außen abstehend Polymerketten 3.2 gebildet sind. Oberhalb einer kritischen Temperatur ("Lower Critical Solution Temperature", LCST), die für Kultivierungsanwendungen typischerweise wenige °C unterhalb von 37 °C gewählt ist, liegen die Polymerketten 3.2 in einem kollabierten Zustand vor (Figur 2A). Bei Abkühlung um eine vorbestimmte Temperaturdifferenz ΔT und Unterschreitung der kritischen Temperatur gehen die Polymerketten 3.2 in einen nicht-kollabierten (gequollenen) Zustand über (Figur 2B). Die Größe der thermoresponsiven Mikrogelpartikel wird mit einem hydrodynamischen Radius beschrieben, der im kollabierten Zustand (R1) geringer als im nicht-kollabierten Zustand (R2) ist. Im nicht-kollabierten Zustand, d.h. unterhalb der kritischen Temperatur können zwischen den Polymerketten 3.2 Kettenbrücken 3.3 bestehen bleiben (Figur 2C), was sich auf die mechanischen Deformationseigenschaften und damit auf die Adhäsionseigenschaften des Substrats 10 für biologische Zellen auswirkt.

In einem praktischen Beispiel ist der Radius R1 der kollabierten Partikel z.B. im Bereich von 2 nm bis 5 µm gewählt. Entsprechend kann ein Radius R2 im nicht-kollabierten Zustand z.B. im Bereich von 4 nm bis 10 µm erreicht werden (von 200 nm bis 420 nm bzw. von 300 nm bis 480 nm in Wu et al. Coll. Poly. Sci. 272 (1994) 467; von 92 nm bis 200 nm, von 42 nm bis 97 nm, von 29 nm bis 65 nm und von 19 nm bis 35 nm in M. Andersson, S.L. Maunu, J. Poly. Sci. B 44 (2006) 3305).

Um die thermoresponsiven Mikrogelpartikel 3 auf der Oberfläche 2 der Zwischenschicht 1 zu immobilisieren, wird zunächst ein Mikrogel hergestellt, das die Partikel als kolloidale Teilchen enthält. Der Radius R1 wird durch die Reaktionsbedingungen bei der Herstellung des Mikrogels eingestellt. Das Mikrogel bildet eine Dispersion, die stabil gelagert werden kann.

Bei der Herstellung des Mikrogels werden vorzugsweise die folgenden Parameter der Partikel eingestellt:
- Polymerkettenlänge,
- Vernetzungsdichte (z.B. Bildung von Kettenbrücken,
- Partikelradius R1 im kollabierten Zustand,
- Partikelradius R2 im nicht-kollabierten Zustand,
- Elastizitätsmodul im kollabierten Zustand,
- Elastizitätsmodul im nicht-kollabierten Zustand, und
- (optional) radialer Gradient der Steifigkeit von innen nach außen.

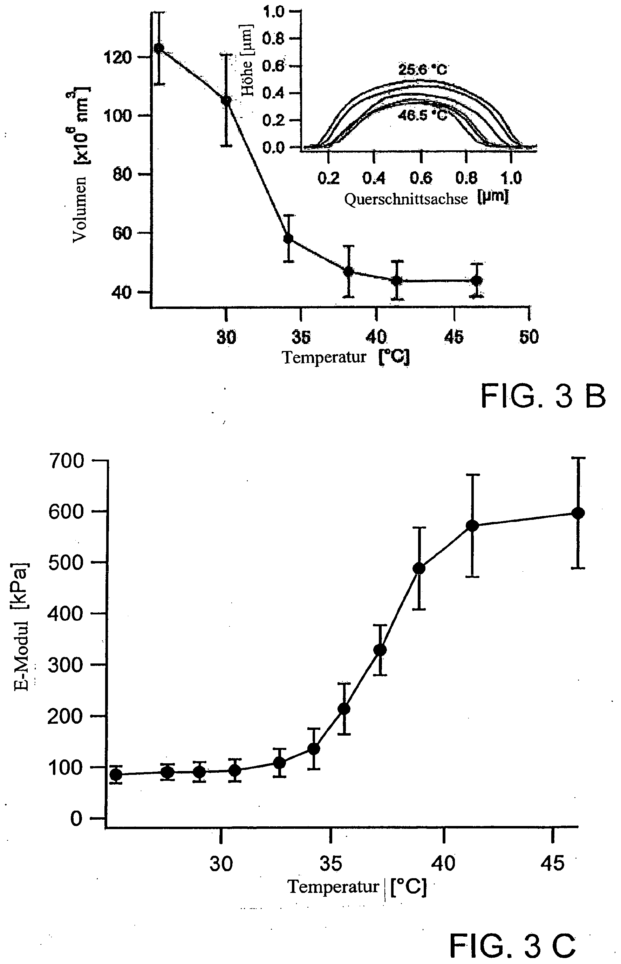

Die Mikrogel-Parameter werden in Abhängigkeit von der Anwendung des Substrats 10 gewählt. Obwohl mit den genannten Eigenschaften ein komplexer Parameterraum aufgespannt wird, ist die Auswahl der konkret zu verwendenden Parameter in Abhängigkeit von den zu kultivierenden Zellen und den zu realisierenden Kultivierungsbedingungen (geometrisch, physikalisch und chemisch) z.B. durch einfache Tests oder durch Verwendung von Tabellenwerten möglich. Die Erfinder haben festgestellt, dass zwischen der Adhäsion biologischer Zellen auf den thermoresponsiven Mikrogelpartikel 3 und deren elastischen Eigenschaften eine starke Korrelation besteht, so dass durch die Auswahl insbesondere von elastischen Eigenschaften des Mikrogels eine Optimierung der Kultivierungsbedingungen möglich ist. So wurde z.B. festgestellt, dass bei einer Änderung des E-Modulus der Mikrogele von 600 kPa (überhalb der LCST) auf 100 kPa (unterhalb der LCST) (siehe Fig. 3C) sehr gute Adhäsions- bzw. Zellablöseeigenschaften bestehen.

Zur Herstellung des Substrats 10 wird das Mikrogel auf die Oberfläche 2 der Zwischenschicht 1 aufgebracht. Es wird eine an sich bekannte Depositionstechnik, wie z.B. Spin-Coating, Eintauchen, Aufsprühen, Stempeln oder Dispensieren, z.B. mit Nadeln oder Dispenserdüsen, verwendet. Die thermoresponsiven Mikrogelpartikel 3, die mit der Zwischenschicht 1 in Kontakt kommen, bilden mit dieser z.B. eine kovalente Bindung. Anschließend wird die beschichtete Oberfläche 2 der Zwischenschicht 1 gewaschen, z.B. mit Wasser, um die überschüssigen, nichtgebundenen Teilchen abzutrennen. Die an der Oberfläche 2 fixierten thermoresponsiven Mikrogelpartikel 3 können, wie in Figur 1 schematisch gezeigt, eine regelmäßige, dichte Packung oder alternativ eine unregelmäßige Packung mit Lücken bilden.

Anschließend kann ein Trocknen der mit den Mikrogelpartikel 3 versehenen Oberfläche 2 der Zwischenschicht 1 vorgesehen sein. Die Trocknung ist jedoch nicht zwingend vorgesehen. Alternativ kann unmittelbar nach dem Abwaschen eine zusätzliche Funktionalisierung oder die Kultivierung biologischer Zellen vorgesehen sein. Des Weiteren kann eine Sterilisierung der freiliegenden Oberfläche der thermoresponsiven Partikel, z.B. durch ionisierende Strahlung (Gammastrahlung) oder Begasung (z.B. mit Ethylenoxid) vorgesehen sein.

Wie in Figur 1 schematisch illustriert, ist mindestens eine biologische Zelle 21 auf der Oberfläche mit thermoresponsiven Mikrogelpartikel 3 im kollabierten Zustand adhärent angeordnet. Durch eine Temperaturabsenkung kann der Phasenübergang der thermoresponsiven Mikrogelpartikel 3 in den nicht-kollabierten Zustand induziert werden, in dem die Härte der Oberfläche mit den thermoresponsiven Mikrogelpartikel 3 im Vergleich zum kollabierten Zustand vermindert ist. Auf der Oberfläche mit der verminderten Härte hat die mindestens eine biologische Zelle 21 eine verminderte Adhäsionsfähigkeit, so dass sie durch das flüssige Kultivierungsmedium über dem Substrat (in Figur 1 nicht gezeigt) abgelöst werden kann.

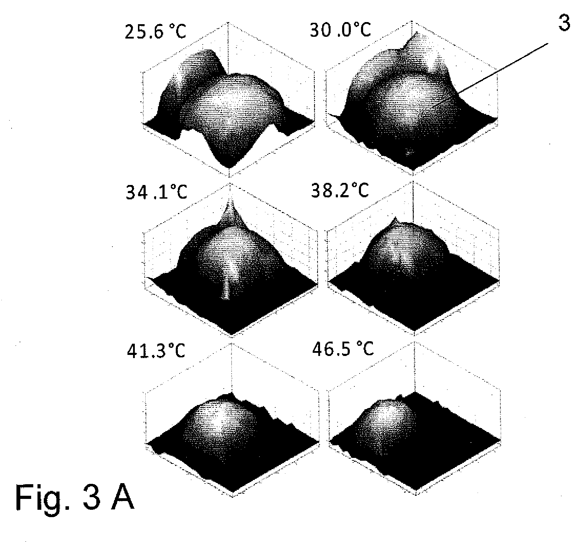

Experimentelle Ergebnisse, die den Phasenübergang von thermoresponsiven Mikrogelpartikeln 3 zeigen, sind beispielhaft in Figur 3 gezeigt. Figur 3A illustriert die mit einem Atomkraftmikroskop gemessene Topographie einzelner Mikrogel-Polymerpartikel 3 (PNIPam) für verschiedene Temperaturen. Quellkurven der Mikrogel-Polymerpartikel 3 im adsorbierten Zustand sind in Figur 3B gezeigt. Die kleine Graphik in Figur 3B zeigt mittlere Höhenprofile im Apex der Mikrogel-Polymerpartikel 3 (Höhe H in Abhängigkeit von Durchmesserkoordinate D, jeweils in µm). Schließlich illustriert Figur 3C die Temperaturabhängigkeit des Young'schen Elastizitäts-Moduls der Polymerpartikel, die aus Messungen mit dem Atomkraftmikroskop abgeleitet wurden. Während bei 37 °C das Elastizitäts-Modul größer als 300 kPa, verringert sich das Elastizitäts-Modul bei 25 °C auf Werte unterhalb von 100 kPa. Gleichzeitig haben die Partikel bei der höheren Temperatur einen geringen Wassergehalt von rund 65 %, während bei 25 °C der Wassergehalt der Polymerpartikel rund 90% ist.

Die experimentellen Ergebnisse zeigen, dass das thermische Ansprechverhalten der Mikrogelpartikel 3, insbesondere das scharfe Temperaturprofil des Phasenübergangs, im adsorbierten Zustand mit dem flüssigen Zustand vergleichbar ist. Experimentelle Tests mit Maus-Fibroblasten haben ergeben, dass die Adhäsion der Fibroblasten auf der Oberfläche in dem Temperaturbereich, in dem der Phasenübergang mit der Änderung des Young-Moduls gemessen wurde, von einem adhärenten Zustand bei Temperaturen oberhalb des Phasenübergangs in einen nicht-adhärenten Zustand bei Temperaturen unterhalb des Phasenübergangs verstellt werden konnte. Oberhalb des Phasenübergangs, z. B. bei 37 °C haben die Zellen eine größere Kontaktfläche mit dem Substrat als bei Temperaturen unterhalb des Phasenübergangs.

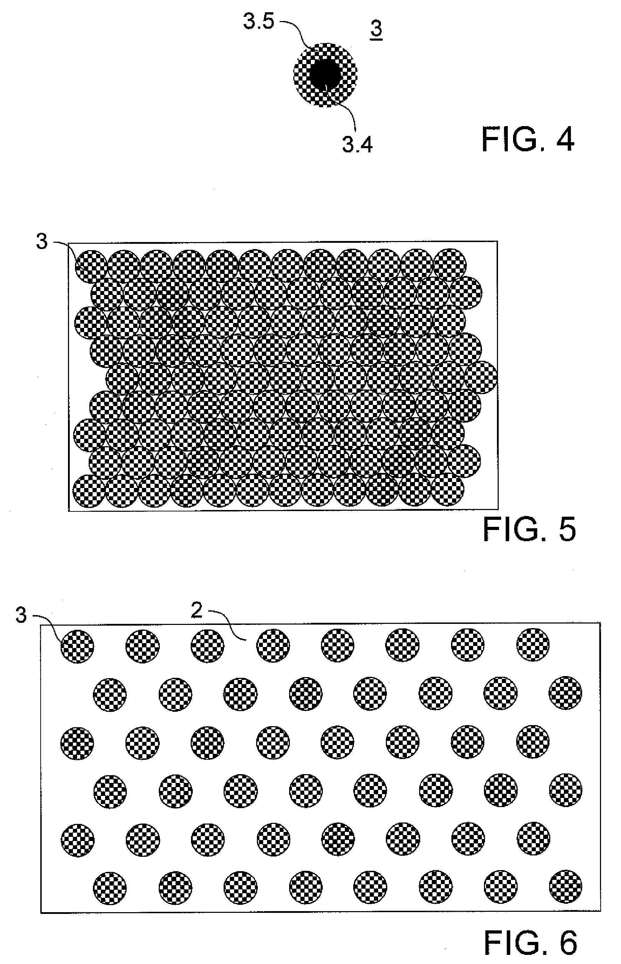

Die Figuren 4 bis 6 zeigen Varianten der Erfindung, welche insbesondere in Abhängigkeit von der konkreten Kultivierungsaufgabe gewählt werden können. So ist gemäß Figur 4 ein thermoresponsiver Mikrogelpartikel 3 mit einer Kern-Schale-Struktur vorgesehen. Der Kern 3.4, z.B. aus Latex, ist unter den Kultivierungsbedingungen und insbesondere bei einer Temperaturänderung unveränderlich. Die Schale 3.5 wird durch das thermoresponsive Polymer, z.B. PNIPam gebildet. Die Herstellung eines Mikrogels zur Bildung von Partikeln mit Kern-Schale-Struktur ist an sich bekannt (siehe T. Hellweg et al. Langmuir 20 (2004) 4330; A. Fernández-Barbero et al. Phys Rev E 66 (2002) 051803/1-10). Die Fixierung von thermoresponsiven Partikeln 3 mit Kern-Schale-Struktur auf der Zwischenschicht und die weitere Behandlung des Substrats erfolgt, wie dies oben unter Bezug auf Figur 1 beschrieben ist.

Die thermoresponsiven Partikel 3 können eine geschlossene (Figur 5) oder eine von Lücken unterbrochene, nicht-geschlossene Monolage (Figur 6) auf der Oberfläche 2 der Zwischenschicht 1 bilden. Die regelmäßige Anordnung der thermoresponsiven Partikel 3 gemäß Figur 5 kann durch Selbstorganisation (Bildung der dichtesten Packung) erzeugt werden. Bei der nicht-geschlossenen Schicht gemäß Figur 6 hingegen kann die regelmäßige Anordnung der thermoresponsiven Partikel 3 durch eine Vorbehandlung der Oberfläche der Zwischenschicht, z.B. mit lokal aufgebrachten Haftvermittler-Inseln erreicht werden. Abweichend von den Figuren 5 und 6 können die thermoresponsiven Partikel 3 unregelmäßige Anordnungen auf der Trägerfläche 2 bilden.

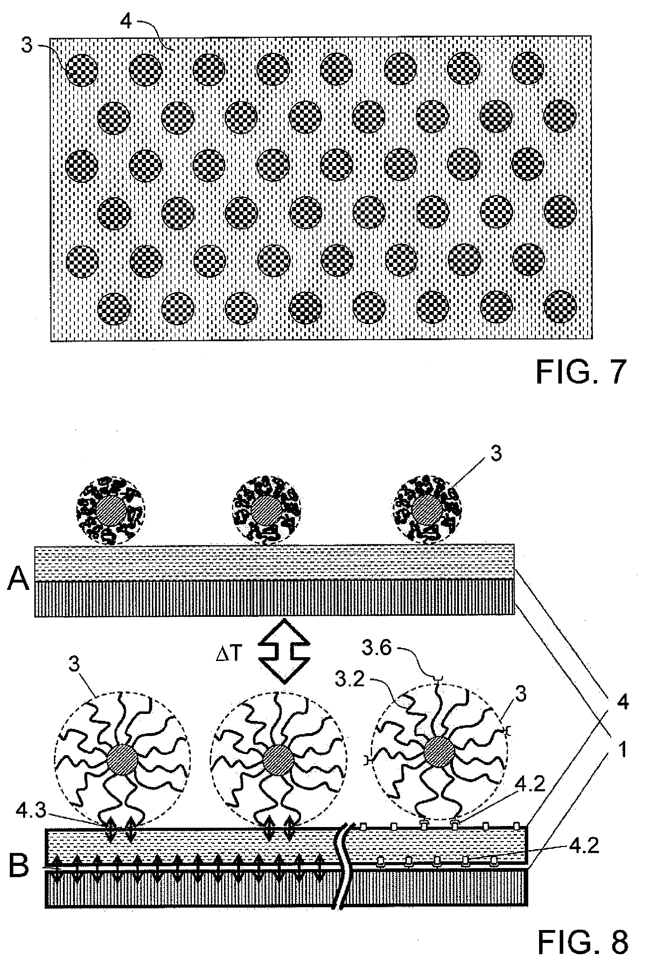

Die Figuren 7 und 8 illustrieren schematisch, dass die Verankerung der thermoresponsiven Partikel 3 auf der Oberfläche der Zwischenschicht verbessert werden kann, wenn auf dieser ein Haftvermittler 4 angeordnet ist. Der Haftvermittler 4 kann die Zwischenschicht komplett bedecken (Figur 7). Figur 8 zeigt einen Ausschnitt des erfindungsgemäßen Substrats 10 mit den thermoresponsiven Polymerpartikeln 3 im kollabierten Zustand (Figur 8A) oberhalb der kritischen Temperatur und im nicht-kollabierten Zustand (Figur 8B) unterhalb der kritischen Temperatur. Figur 8B illustriert des Weiteren schematisch die zwischen dem Haftvermittler 4 mit einerseits der Zwischenschicht 1 und andererseits den thermoresponsiven Partikeln 3 vorgesehenen Bindungsvarianten. So können an den freien Enden der Polymerketten 3.2 der thermoresponsiven Polymerpartikel 3 Bindungsplätze 3.6 für eine kovalente oder biospezifische Bindung 4.1 mit dem Haftvermittler 4 vorgesehen sein. Die Bindungsplätze 3.6 können bei der Herstellung des Mikrogels gebildet werden. Die kovalenten Bindungen basieren auf z.B. Epoxid-, Carboxy-, Amino-, Hydrazid-, Thiol- oder Maleimid-Verbindungen an den Enden der Polymerketten 3.2. Zur Bildung der kovalenten oder biospezifische Bindung 4.1 ist die Haftvermittler-Schicht 4 entsprechend mit Bindungsplätzen 4.2 ausgestattet, die mit den Bindungsplätzen 3.6 der thermoresponsiven Partikel 3 reagieren. Gleichzeitig bilden die Bindungsplätze 4.2 mit der Zwischenschicht 1 kovalente oder biospezifische Bindungen aus. Die biospezifischen Bindungen können insbesondere durch Rezeptor-Ligand-Bindungen, wie z.B. zwischen Streptavidin und Biotin gebildet werden.

Im linken Teil von Figur 8B ist schematisch illustriert, dass die Wirkung des Haftvermittlers 4 auf einer unspezifischen Wechselwirkung 4.3 einerseits mit den thermoresponsiven Partikeln 3 und andererseits mit der Zwischenschicht 1 beruhen kann.

Der Haftvermittler 4 umfasst z.B. eine Biotin-Schicht mit einer Dicke von 1 nm bis 1 µm (J. Spinke et al. J. Chem. Phys. 99 (1993) 7012; J.D. Hong et al. Progr. Colloid Polym. Sci 93 (1993) 98; W. Zao et al. Electroanal. 18 (2006) 1737). Die Schicht wird mit an sich bekannten Verfahren, wie z.B. Spin-Coating oder Selbstassemblierung aus der Lösung, auf der Oberfläche des Substratkörpers 1 gebildet.

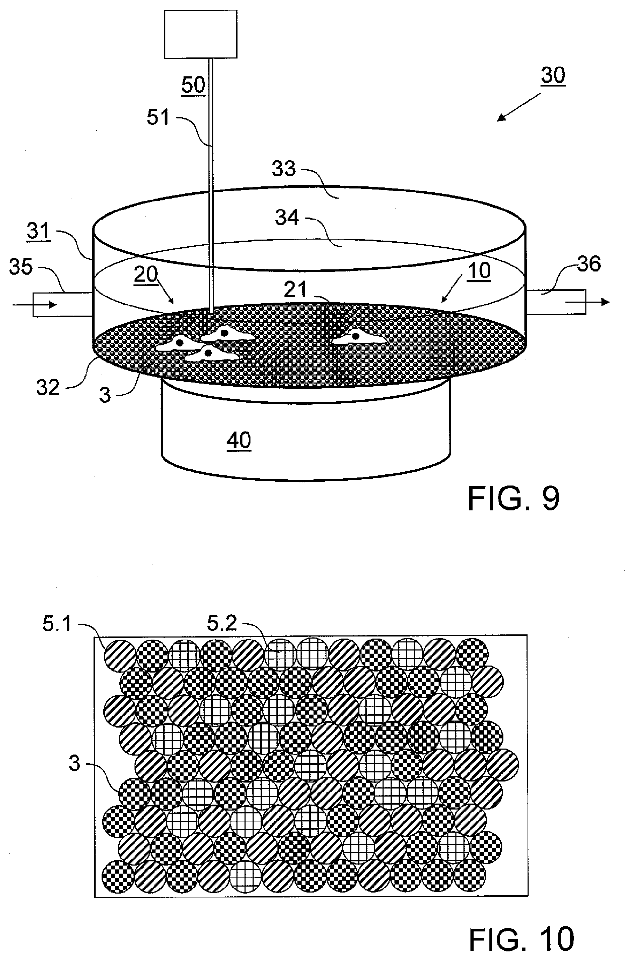

Das erfindungsgemäße Substrat 10 kann Teil einer Kultivierungseinrichtung 30 sein, wie beispielhaft in Figur 9 illustriert ist. Die Kultivierungseinrichtung 30 umfasst ein Kultivierungsgefäß 31 mit einem Boden 32 und einer umlaufenden Seitenwand 33. Das Kultivierungsgefäß 31 ist zur Aufnahme eines flüssigen Kultivierungsmediums 34 vorgesehen, das durch eine Zufuhrleitung 35 in das Kultivierungsgefäß 31 eingeführt und durch eine Auslassleitung 36 aus dem Kultivierungsgefäß 31 entfernt werden kann. Das erfindungsgemäße Substrat 10 ist auf dem Boden 32 angeordnet. Alternativ bildet der Boden 32 das Substrat 10. Auf der zum Inneren des Kultivierungsgefäßes 31 weisenden Seite des Substrats 10 sind freiliegend die thermoresponsiven Partikel 3 angeordnet. Auf dem Substrat 10 befinden sich biologische Zellen 20, 21.

Figur 9 illustriert des Weiteren schematisch eine Temperatureinstelleinrichtung 40 und eine Manipulatoreinrichtung 50. Mit der Temperatureinstelleinrichtung 40 kann die Temperatur des Substrats 10 oder von Teilbereichen des Substrats 10 gezielt von einer Temperatur oberhalb bis zu einer Temperatur unterhalb der kritischen Temperatur des Phasenübergangs der thermoresponsiven Mikrogelpartikel 3 eingestellt werden. Die Temperatureinstelleinrichtung umfasst z.B. eine Heizeinrichtung, wie z. B. eine Widerstandsheizung, oder eine Kombination aus einer Heizeinrichtung und einer Kühleinrichtung, wie z. B. eine Peltier-Kühlung. Die Manipulatoreinrichtung 50 umfasst z.B. eine Zufuhrleitung 51, durch die eine Zellsuspension in das Kultivierungsgefäß 31 gespült werden kann.

Des Weiteren kann die Kultivierungseinrichtung 30 mit einer Beobachtungseinrichtung, z.B. einem Mikroskop, und einer Messeinrichtung, z.B. einem Temperatursensor (nicht dargestellt) ausgestattet sein.

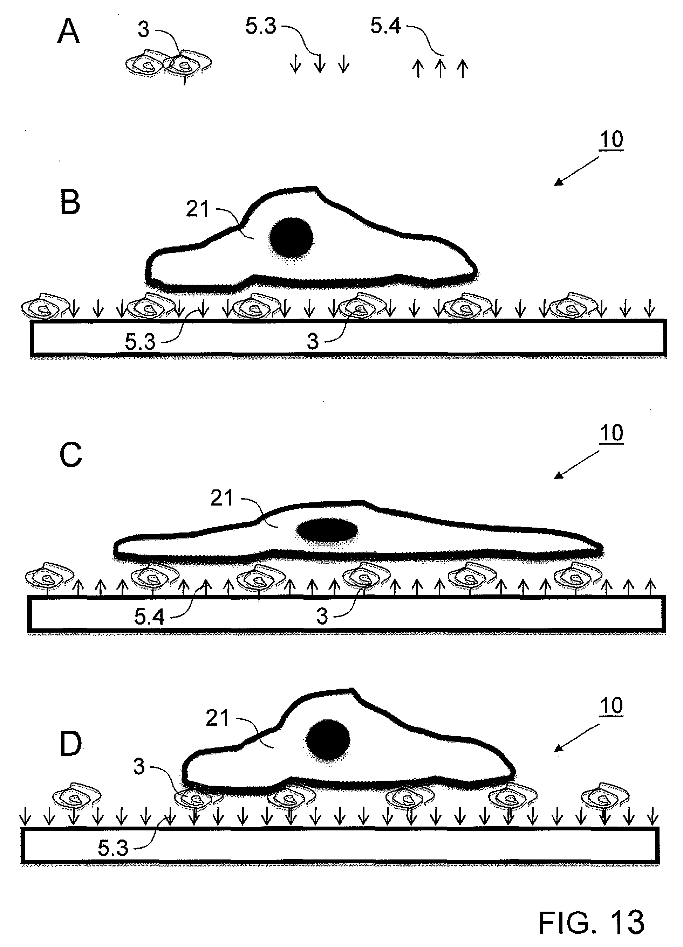

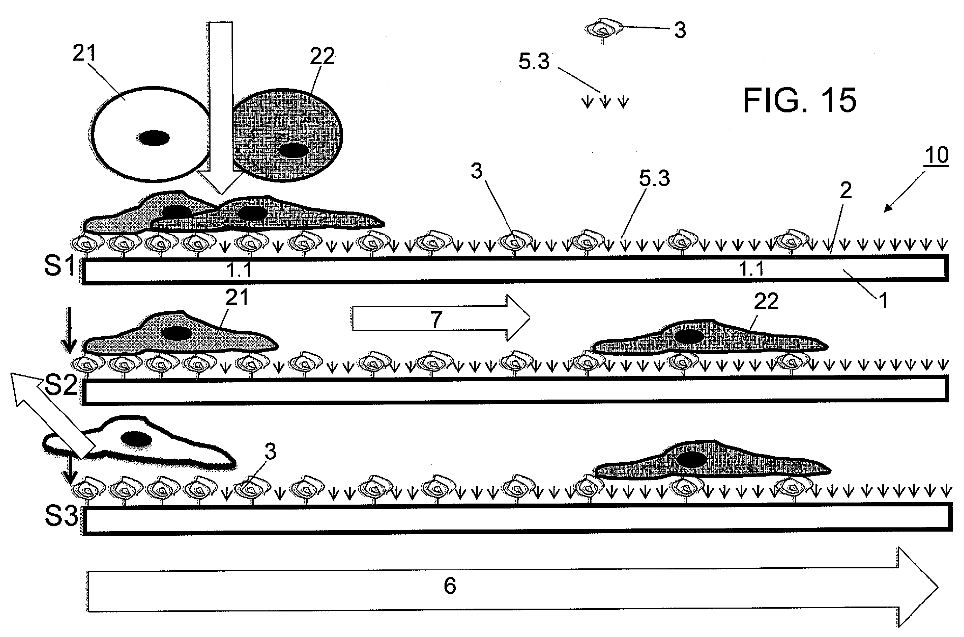

Die Figuren 10 bis 17 illustrieren verschiedene Varianten der Funktionalisierung eines erfindungsgemäßen Substrats mit mindestens einer Modulatorsubstanz, die mit Modulatorpartikeln (z.B. Figuren 10 bis 12) und/oder als Modulatorschicht (z.B. Figuren 13, 15) eine Zwischenschicht bilden bzw. Bestandteil einer Zwischenschicht sein kann und/oder Bestandteil der die Mikrogelpartikel enthaltenden Schicht M sein kann.

Allgemein umfasst die Modulatorsubstanz eine einzelne chemische Substanz oder eine Zusammensetzung chemischer Substanzen, zu der die biologischen Zellen eine im Vergleich zu den thermoresponsiven Polymerpartikeln veränderte Adhäsionsfähigkeit aufweisen (Beispiele siehe oben) und/oder mit der in den biologischen Zellen zelluläre Reaktionen induzierbar sind.

Substanzen, welche zelluläre Reaktionen auslösen, sind allgemein Substanzen, welche durch Bindung an Oberflächenrezeptoren der biologischen Zellen z.B. eine verstärkte Adhäsion, eine Migration (Zellwanderung), eine Differenzierung (insbesondere Stammzelldifferenzierung), eine Änderung des Aktivierungsstatus oder eine Änderung der Malignität bewirken. Derartige Substanzen sind z.B.:
- Chemokine wie z.B. FGF induzieren Chemotaxis
- Osteonektin induziert Differenzierung von Stammzellen in Herzmuskelzellen.

Vorteilhafterweise ermöglicht die Kombination verschieden wirkender Modulatorsubstanzen die gezielte Einstellung vorbestimmter physikalischer oder chemischer Oberflächeneigenschaften. Da die mindestens eine Modulatorsubstanz bei der Herstellung des Mikrogels der Dispersion kolloidaler Teilchen des thermoresponsiven Polymers zugesetzt werden kann, lassen sich die thermoresponsiven Polymerpartikel und die mindestens eine Modulatorsubstanz wie Module frei kombinieren. Die Oberfläche des erfindungsgemäßen Substrats kann wie ein modularer Baukasten gestaltet werden.

Bei dem schematisch in Figur 10 gezeigten Beispiel sind thermoresponsive Mikrogelpartikel 3, Kunststoffpartikel, die mit zellanziehenden Molekülen beschichtet sind (adhäsionssteigernde Modulatorpartikel 5.1) und Kunststoffpartikel, die mit zellabstoßenden Molekülen beschichtet sind (adhäsionsmindernde Modulatorpartikel 5.2) kombiniert. Die Modulatorpartikel 5.1, 5.2 haben einen Durchmesser, der z.B. im Bereich 50 nm bis 1 µm gewählt ist.

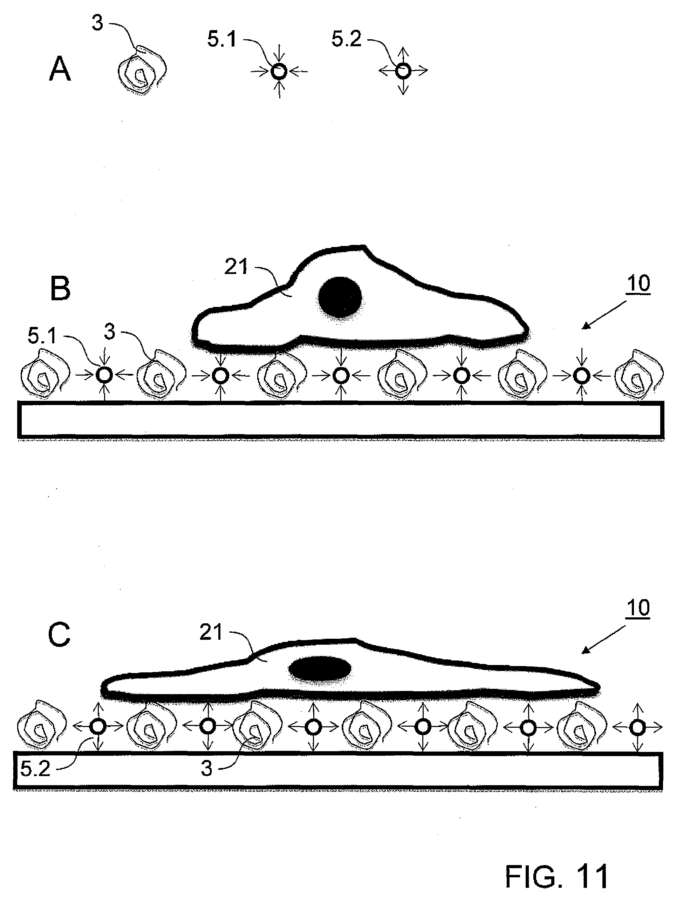

Figur 11 illustriert schematisch die verschiedenen Wirkungen der Kombination thermoresponsiver Partikel 3 mit adhäsionssteigernden Modulatorpartikeln 5.1 und adhäsionsmindernden Modulatorpartikeln 5.2 (symbolisiert in Figur 11A). Gemäß Figur 11B bewirken die adhäsionssteigernden Modulatorpartikel 5.1 durch eine relativ hohe Anzahl von Bindungsplätzen für die adhärente Anbindung der Zelle 21, dass eine relativ kleine Kontaktfläche zwischen der Zelle 21 und der Substratoberfläche gebildet wird. Mit der kleineren Kontaktfläche berührt die Zelle 21 relativ wenige thermoresponsive Partikel 3, so dass deren Wirkung bei einem temperaturabhängigen Phasenübergang vermindert wird. Im Ergebnis wird eine starke Bindung der Zelle 21 zum Substrat 10 erzielt.

Gemäß Figur 11C bewirken adhäsionsmindernde Modulatorpartikel 5.2 einen gegenteiligen Effekt. Die Zelle 21 wird auf der Oberfläche des Substrats 10 ausgebreitet, um Bindungsplätze für die Adhäsionskontakte der Zelle 21 zu finden. Entsprechend erhält die Zelle 21 Kontakt mit einer relativ großen Anzahl thermoresponsiver Partikel 3. Somit wirkt sich ein Phasenübergang der thermoresponsiven Partikel 3 stärker als bei den adhäsionssteigernden Modulatorpartikeln 5.1 (Figur 11B) aus. Die Adhäsion der Zelle 21 auf der Oberfläche des Substrats 10 wird vermindert.

Durch eine Einstellung der quantitativen Mischungsverhältnisse der thermoresponsiven Polymerpartikel 3 mit mindestens einem Typ der Modulatorpartikel 5.1, 5.2 in der Dispersion zur Herstellung des Mikrogels können somit vorteilhafterweise die Adhäsionseigenschaften (Anhaft- oder Ablösungsparameter) der Substratoberfläche über einen weiten Bereich variiert werden, wobei der thermoresponsive Charakter der Oberfläche erhalten bleibt und die Oberfläche für einen Zelltyp oder mehrere Zelltypen optimale Adhäsionseigenschaften besitzt. Vorteilhafterweise können die Mischungsverhältnisse in Trägerlösungen zur Herstellung der Dispersionen einfach durch Einwiegen erzeugt werden. Die Verwendung des Mikrogels, welches die Teilchen des thermoresponsiven Polymers und die Modulatorpartikel enthält, ermöglicht die gemeinsame Übertragung auf das Substrats in einem einzigen Depositionsschritt.

Die in der schematischen Illustration von Figur 11 gewählten Größenverhältnisse einerseits der biologischen Zelle 21 und andererseits der Partikel 3, 5.1 und 5.2 sind aus zeichnungstechnischen Gründen gewählt. Im Unterschied zur Illustration können erheblich geringere Partikelgrößen, z.B. bis 50 nm oder darunter, oder auch größere Partikel, z.B. 10 µm verwendet werden.

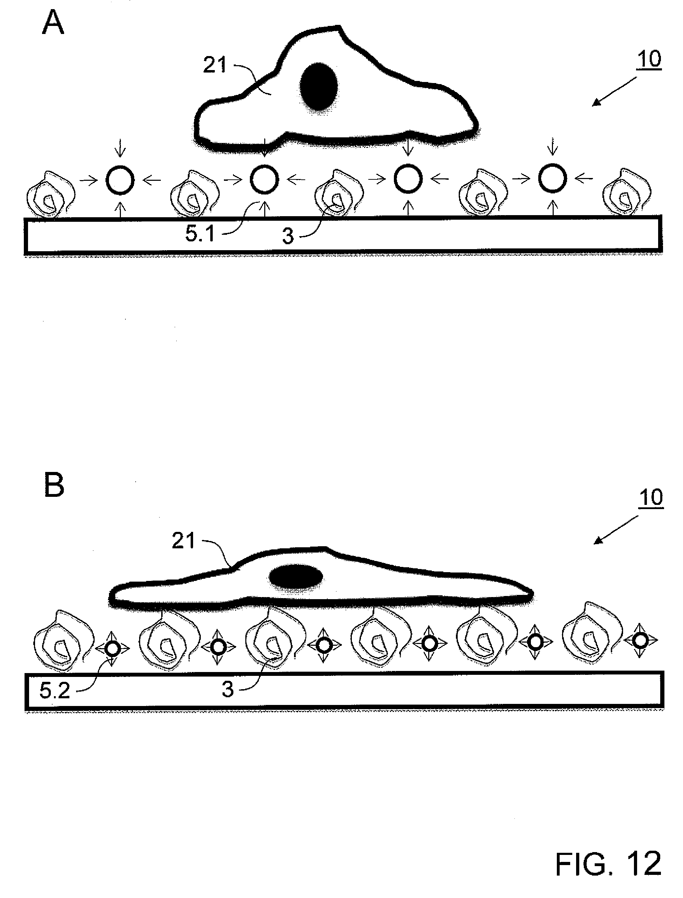

Gemäß einer weiteren Variante der Erfindung können auf der Oberfläche des Substrats 10 Partikel mit verschiedenen Größen kombiniert werden, wie beispielhaft in Figur 12 illustriert ist. Beispielsweise können die adhäsionssteigernden Modulatorpartikel 5.1 einen größeren Radius als die thermoresponsiven Polymerpartikel 3 haben (Figur 12A). In diesem Fall wird die adhäsionssteigernde Wirkung der Modulatorpartikel 5.1 verstärkt, da diese im Vergleich zu den thermoresponsiven Polymerpartikeln 3 für die Zelle 21 stärker zugänglich sind. Dazu im Gegensatz wird gemäß Figur 12B mit adhäsionsmindernden Modulatorpartikeln 5.2, deren Radius geringer als der Radius der thermoresponsiven Polymerpartikel 3 ist, die Wirkung der Modulatorpartikel 5.2 vermindert. Weitere Kombinationen, wie z.B. kleinere adhäsionssteigernde Modulatorpartikel 5.1 und/oder größere adhäsionsmindernde Modulatorpartikel 5.2 sind ebenfalls möglich.

Im Ergebnis können nicht nur die Adhäsionseigenschaften der Oberfläche eingestellt, sondern auch eine bestimmte Körnigkeit der Oberfläche bereitgestellt werden. Die Bereitstellung einer körnigen Oberfläche bedeutet, dass eine Oberflächentopologie mit Erhöhungen und Vertiefungen erzeugt wird. Vorteilhafterweise kann die Körnigkeit der Oberfläche an typische Dimensionen des Adhäsionsmusters eines bestimmten Zelltyps angepasst werden (Humane und Bovin Kapillarendothel-Zellen - C.S. Chen et al., Science 276 (1997) 1425 und C2C12-Muskelzellen - U. Joos et al. Eur. J. Cell Bio. 85 (2006) 225).

Bei den in den Figuren 10-12 dargestellten Substraten liegen die Modulatorpartikel gemeinsam mit den thermoresponsiven Mikrogelpartikeln in der Schicht M vor, die wiederum auf der obersten Zwischenschicht angebracht ist.

Figur 13 illustriert eine weitere Variante der Erfindung, bei der die mindestens eine Modulatorsubstanz in Kombination mit den thermoresponsiven Polymerpartikeln 3 als adhäsionssteigernde Modulatorschicht 5.3 oder als adhäsionsmindernde Modulatorschicht 5.4 bereitgestellt wird (Figur 13A). Auch in diesem Fall wird vorteilhafterweise eine modulare Gestaltung der Oberfläche des Substrats 10 durch eine Überlagerung der Wechselwirkung der Zelle 21 mit den verschiedenen Komponenten erzielt. Im Unterschied zu der Verwendung von Modulatorpartikeln wird die mindestens eine Modulatorsubstanz nicht dem Mikrogel zugesetzt, sondern in einem zusätzlichen Depositionsschritt durch eine Beschichtung der Trägerfläche des Substratkörpers oder einer Zwischenschicht bereitgestellt.

Figur 13B illustriert die Wirkung einer adhäsionssteigernden Modulatorschicht 5.3, die analog zu Figur 11B eine verkleinerte Kontaktfläche der Zelle 21 und damit einer verminderte Wirkung der thermoresponsiven Polymerpartikel 3 ergibt. Dazu im Gegensatz liefert die adhäsionsmindernde Modulatorschicht 5.4 gemäß Figur 13C eine Ausbreitung der Zelle 21 und damit eine verstärkte Wirkung der thermoresponsiven Polymerpartikel 3.

Während bei den Varianten der Figuren 13B und 13C die thermoresponsiven Polymerpartikel 3 durch eine der oben beschriebenen Verbindungstypen an die benachbarte Zwischenschicht gekoppelt ist, besteht gemäß Figur 13D alternativ die Möglichkeit, die thermoresponsiven Polymerpartikel 3 mit der adhäsionssteigernden Modulatorschicht 5.3 zu verbinden. In diesem Fall erfüllt die adhäsionssteigernde Modulatorschicht 5.3 eine Doppelfunktion als Haftvermittler (siehe oben, Figuren 7, 8) und als Modulatorsubstanz.

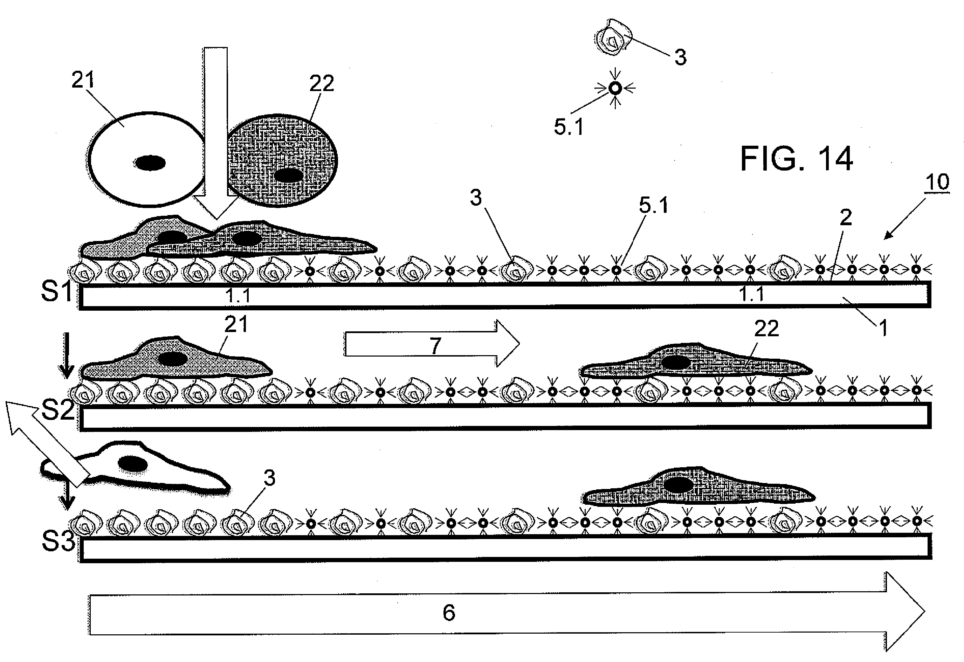

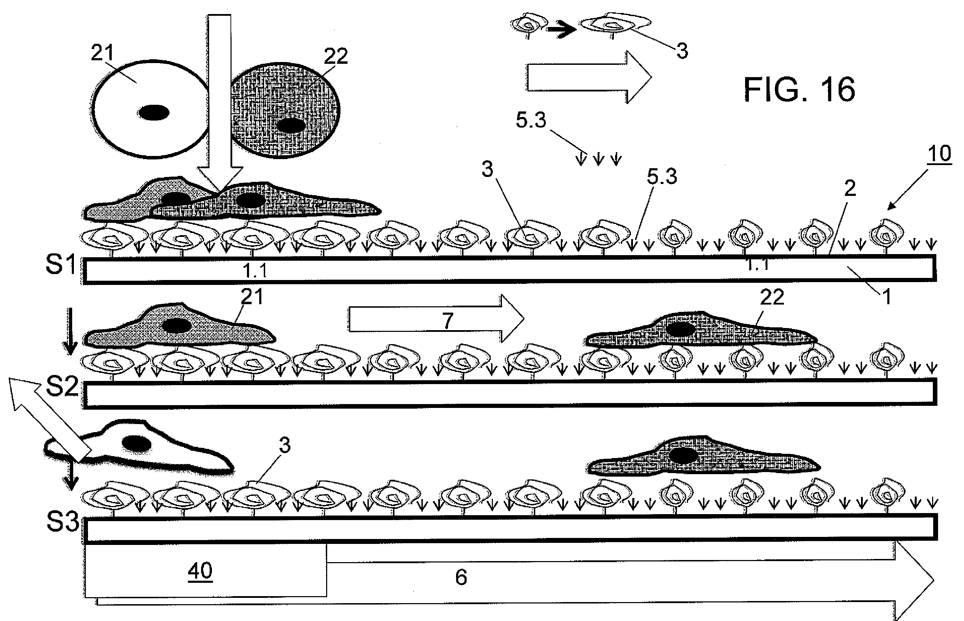

Die Figuren 14, 15 und 16 illustrieren Ausführungsformen der Erfindung, bei denen die thermoresponsiven Mikrogelpartikel und die Modulatorsubstanz gemeinsam die Schicht M bilden und auf der Oberfläche 2 der benachbarten Zwischenschicht 1 mit mindestens einem Dichtegradienten angeordnet sind. Diese Ausführungsformen sind insbesondere für die Manipulation biologischer Zellen bei der Kultivierung in Kokulturen von Vorteil. Durch die Bildung von Dichtegradienten lassen sich die Oberflächeneigenschaften des Substrats so modifizieren, dass eine Migration (Zellwanderung) der adhärenten Zellen in Abhängigkeit vom Zelltyp induziert wird. Hierzu kann ein Konzentrationsgradient (Figuren 14, 15) und/oder ein Funktionsgradient (Figur 16) von mindestens einer Modulatorsubstanz mit chemotaktischen Eigenschaften vorgesehen sein.

Gemäß Figur 14 sind auf der Oberfläche 2 der Zwischenschicht 1 thermoresponsive Mikrogelpartikel 3 und adhäsionssteigernde Modulatorpartikel 5.1 so angeordnet, dass in einem ersten Teilbereich 1.1 eine höhere Flächendichte der thermoresponsiven Partikel 3 im Vergleich zu den adhäsionssteigernden Modulatorpartikeln 5.1 und in einem zweiten Teilbereich 1.2 umgekehrt eine geringere Dichte der thermoresponsiven Partikel 3 im Vergleich zu den adhäsionssteigernden Modulatorpartikeln 5.1 erzeugt wird. Im Ergebnis wird ein Dichtegradient 6 gebildet, der sich entlang der Trägerfläche 2 durch eine zunehmende Flächendichte der adhäsionssteigernden Modulatorpartikel 5.1 oder eine abnehmende Flächendichte der thermoresponsiven Partikel 3 auszeichnet. Der Dichtegradient 6, der in Figur 14 schematisch illustriert ist, kann in der Praxis stufenweise durch die Deposition von Mikrogelen mit verschiedenen Zusammensetzungen auf verschiedenen Teilbereichen der Zwischenschicht 1 erzeugt werden.

Gemäß Figur 14 ist zur Kultivierung biologischer Zellen 21 von einem ersten, interessierenden Zelltyp gemeinsam mit Feeder-Zellen 22 die Bildung der Kokultur in dem ersten Teilbereich 1.1 mit einem hohen Anteil der thermoresponsiven Partikel 3 vorgesehen (Schritt S1). Nach der Kultivierung, die z.B. eine Differenzierung der Zellen 21 umfasst, wandern die Feeder-Zellen 22 unter der spezifischen Wirkung der adhäsionssteigernden Modulatorpartikel 5.1 aus dem ersten Teilbereich 1.1 heraus (Migration 7, Schritt S2). Anschließend erfolgt die Ablösung der interessierenden Zellen 21, indem durch eine Temperaturänderung des Substrats 10 der Phasenübergang der thermoresponsiven Partikel 3 induziert und im Teilbereich 1.1 die Adhäsionsfähigkeit für biologische Zellen 21 erheblich vermindert wird. Die Zellen 21 können dann, z.B. mit einer Manipulationseinrichtung, wie sie in Figur 9 gezeigt ist, vom Substrat 10 entfernt werden.

Um Zellen zelltypspezifisch zur Migration anzuregen, stehen eine Reihe von Modulatorsubstanzen zur Verfügung. Beispielsweise wirkt fMLP ausschließlich auf die Migration von HL 60 Leukämie-Zellen, während andere Zelltypen unbeeinflusst bleiben.

Das in Figur 14 schematisch gezeigte Prinzip der selektiven Migration 7 eines Zelltyps aus einer Mischung von adhärenten Zellen lässt sich entsprechend auf die Mischung von mehr als zwei Zelltypen verallgemeinern, wobei die adhäsionssteigernde Modulatorsubstanz so gewählt ist, dass mindestens ein Zelltyp aus der Mischung auswandert oder mindestens ein Zelltyp unverändert bleibt und keine Migration zeigt. Vorteilhafterweise können damit kleine Zellproben mit geringen Zellzahlen insbesondere unterhalb von 10⁵ Zellen nach der Kokultivierung getrennt werden.

Die Trennung von Mischungen verschiedener Zelltypen ist nicht auf die Verwendung von adhäsionssteigernden Modulatorpartikeln beschränkt. Alternativ oder zusätzlich können thermoresponsive Partikel 3 mit adhäsionssteigernden Modulatorschichten 5.3 kombiniert werden, wie schematisch in Figur 15 illustriert ist. Es wird ein Dichtegradient 6 zwischen einem ersten Teilbereich 1.1 der Zwischenschicht 1 mit einer erhöhten Flächendichte der thermoresponsiven Partikel 3 und einem weiteren Teilbereich 1.2 der Zwischenschicht 1 mit einer erhöhten Flächendichte der adhäsionssteigernden Modulatorschicht 5.3 gebildet. Analog zu Figur 14 erfolgt zunächst die Ablage und Kokultur der interessierenden biologischen Zellen 21 gemeinsam mit Feeder-Zellen 22 (Schritt S1), anschließend die zelltypspezifische Migration 7 der Feeder-Zellen 22 aus dem Zellgemisch heraus (Schritt S2) und schließlich die Ablösung der interessierenden Zelle 21 durch den temperaturinduzierten Phasenübergang der thermoresponsiven Partikel 3 (Schritt S3).

Für die Ablösung der Zellen 21 stehen verschiedene Optionen zur Verfügung. Gemäß den Figuren 15 und 16 kann die Temperatur des gesamten Substrats 10 unter die kritische Temperatur (LCST) abgesenkt werden. Dies ist insbesondere dann möglich, wenn die adhärent bleibenden Zellen 22 im Teilbereich 1.2 vom Phasenübergang der thermoresponsiven Partikel 3 unbeeinflusst bleiben. Alternativ kann eine lokale Verminderung der Temperatur vorgesehen sein, wie schematisch in Figur 16 illustriert ist. Bei dieser Ausführungsform der Erfindung ist die Temperatureinstelleinrichtung 40 (siehe auch Figur 9) lokal auf den Teilbereich 1.1 der Zwischenschicht 1 wirkend angeordnet. In diesem Fall kann der Phasenübergang der thermoresponsiven Partikel 3 lokal beschränkt im Teilbereich 1.1 induziert werden, während die thermoresponsiven Partikel in anderen Teilbereichen unverändert bleiben.

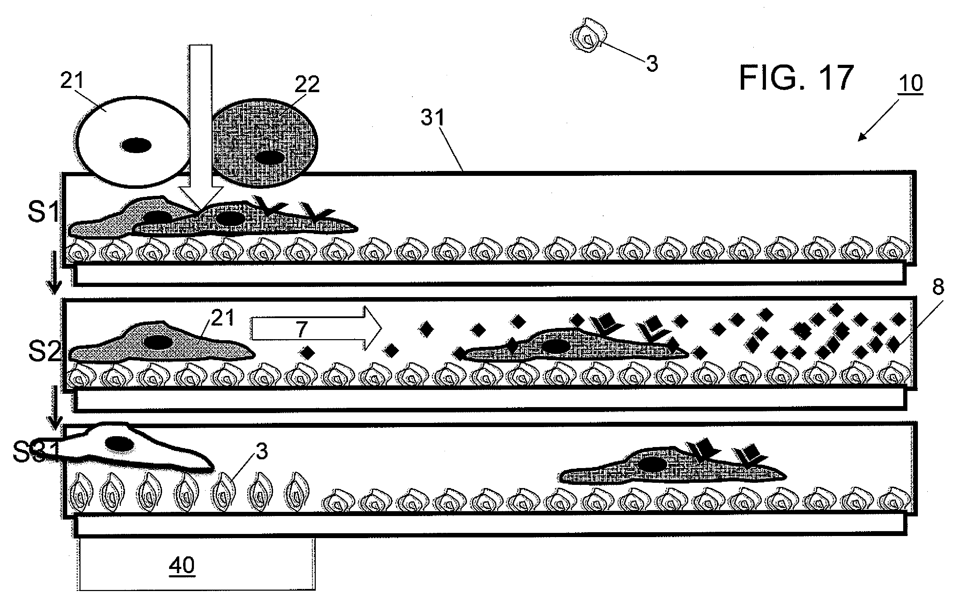

Die zelltypspezifische Migration auf der Substratoberfläche erfordert jedoch nicht zwingend einen Dichtegradienten. Alternativ oder zusätzlich können chemotaktisch wirkende Substanzen 8 dem Kultivierungsmedium zugesetzt werden, wie schematisch in Figur 17 illustriert ist.

Gemäß Figur 17 wird ein erfindungsgemäßes Substrat 10 mit einer Zwischenschicht 1 verwendet, auf der die thermoresponsiven Partikel 3 homogen verteilt angeordnet sind. Nach der Kokultivierung der Zellen 21, 22 auf dem Substrat 10 (Schritt S1) erfolgt der Zusatz chemotaktisch wirkender Substanzen 8 in das Kultivierungsmedium, so dass eine Migration 7 der Zellen induziert wird. Durch Auswahl der chemotaktisch wirkenden Substanz 8 kann die Migration 7 zelltypspezifisch induziert werden. Beispielsweise bewirkt fMLP ausschließlich eine Migration von HL 60 Leukämie-Zellen, während Zellen von Zelllinien, die aus gesundem Gewebe gewonnen wurden, unbeeinflusst bleiben.

Zur Ablösung der interessierenden Zellen 21 erfolgt bei Schritt S3 eine lokal begrenzte Temperaturabsenkung. Die thermoresponsiven Partikel 3 zeigen den Phasenübergang in dem nicht-kollabierten Zustand, so dass die Zelle 21 abgelöst werden kann.

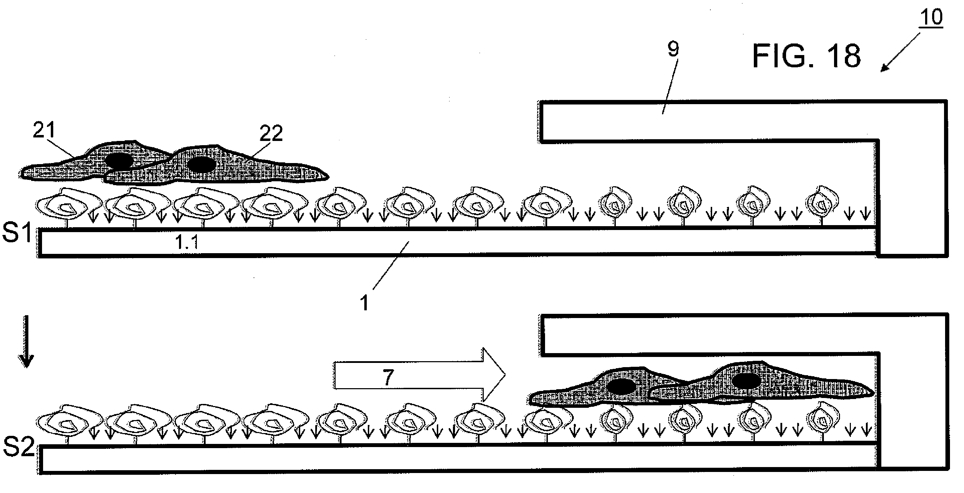

Figur 18 zeigt eine weitere Variante der Erfindung, bei der das Substrat 10 mit einer Kultivierungskavität 9 ausgestattet ist. Die Kultivierungskavität 9 kann zur *in-vitro-*Simulation von Differenzierungsvorgängen in Stammzellnischen verwendet werden. So werden im biologischen Organismus Stammzellen in Kavitäten mit einer vorbestimmten biochemischen und/oder zellulären Auskleidung vorgehalten (siehe David T. Scadden, Nature 441 (2006) 1075; M.C. Dusseiller et al. Biointerphases 1 (2006) P1) und einer Differenzierung unterzogen. Ein Beispiel einer derartigen Stammzellnische sind Haarfollikeln.

Mit der Kultivierungskavität 9 des erfindungsgemäßen Substrats 10 wird eine Mikroumgebung für biologische Zellen 21 geschaffen, in der die Bedingungen im Organismus nachgebildet werden. Herkömmliche Zellmanipulationstechniken zur Auskleidung künstlicher Kultivierungskavitäten mit biologischen Zellen erfordern die Verwendung optischer Pinzetten oder dielektrophoretisch wirkender Elemente. Dies ist hinsichtlich des Geräteaufwands und der komplexen Verfahren nachteilig.

Dieses Problem wird mit dem erfindungsgemäßen Substrat gemäß Figur 18 dadurch gelöst, dass Zellen gezielt in die Kultivierungskavität 9 einwandern.

Gemäß Schritt S1 in Figur 18 wird ein Zellgemisch aus biologischen Zellen 21, 22, die in der Kultivierungskavität 9 angeordnet werden sollen, in einem ersten Teilbereich 1.1 der Zwischenschicht 1 aufgebracht und ggf. kultiviert. Bei einem zweiten Schritt S2 erfolgt die gezielte Migration der Zellen 21, 22 in die Kultivierungskavität 9, wobei einer der oben genannten Mechanismen, z.B. ein Dichtegradient und/oder eine aus dem Kulturmedium wirkende chemotaktische Substanz verwendet werden.

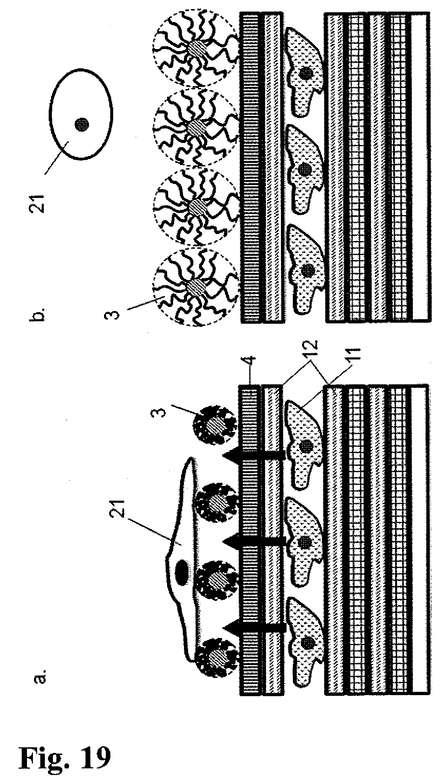

Fig. 19 zeigt eine bevorzugte Ausführungsform des erfindungsgemäßen Substrats, bei der zumindest eine Zwischenschicht 11 biologische Zellen umfasst. Diese Zwischenschicht 11 kann weitere Komponenten enthalten oder alternativ ausschließlich aus den biologischen Zellen gebildet sein. Bevorzugt befindet sich zwischen dieser, die biologischen Zellen enthaltenden Schicht 11 und der die Mikrogelpartikel 3 enthaltenden Schicht M zumindest eine weitere Zwischenschicht 12, die bevorzugt aus einem zellkompatiblen Material gefertigt ist. Bevorzugt grenzen sowohl die Unterseite als auch die Oberseite der Schicht 11 an eine benachbarte Zwischenschicht aus zellkompatiblem Material.

Zusätzlich zu der die biologischen Zellen enthaltenden Zwischenschicht 11 und der Zwischenschicht bzw. den Zwischenschichten 12 aus zellkompatiblem Material kann das Substrat weitere Zwischenschichten aufweisen, wie z.B. eine Haftvermittlerschicht 4.

Bei den in der Schicht 11 vorliegenden Zellen handelt es sich bevorzugt um Zellen, die zur Abgabe biologisch aktiver Substanzen fähig sind und diese biologisch aktiven Substanzen eine spezifische Wechselwirkung mit den auf der Mikrogelpartikelschicht vorliegenden biologischen Zellen 21, den Zielzellen, eingehen können. Die biologisch aktiven Substanzen können z.B. spezifische zelluläre Reaktionen bei den Zielzellen 21 auslösen.

Substanzen, welche zelluläre Reaktionen auslösen, sind allgemein Substanzen, welche durch Bindung an Oberflächenrezeptoren der biologischen Zellen z.B. eine verstärkte Adhäsion, eine Migration (Zellwanderung), eine Differenzierung (insbesondere Stammzelldifferenzierung), eine Änderung des Aktivierungsstatus oder eine Änderung der Malignität bewirken.

In der in Figur 19 dargestellten Ausführungsform liegen die Feederzellen und die Zielzellen räumlich separiert in getrennten Schichten vor. Dies erleichtert später die selektive Abtrennung der Zielzellen 21 vom Substrat.

Das zellkompatible Material der Schicht 12 wird bevorzugt so ausgewählt, dass nicht nur eine ausreichende Zellkompatibilität gewährleistet ist, sondern auch eine ausreichend schnelle Diffusion der von den Feederzellen abgesonderten, biologisch aktiven Substanz durch diese Schicht 9 sicher gestellt ist. Geeignete zellkompatible Zwischenschichten können zum Beispiel Polysacchariden, Poly(vinylpyrrolidon) und/oder Poly(diallyldimethylammoniumchlorid) umfassen.

Die in der vorstehenden Beschreibung, den Zeichnungen und den Ansprüchen offenbarten Merkmale der Erfindung können sowohl einzeln als auch in Kombination für die Verwirklichung der Erfindung in ihren verschiedenen Ausgestaltungen von Bedeutung sein.

## Patentansprüche

1. Ein Substrat, insbesondere zur Aufnahme biologischer Zellen, umfassend:
- einen Substratkörper, der eine Trägerfläche aufweist,
- eine oder mehrere Zwischenschichten Z, die auf der Trägerfläche aufgebracht ist/sind, wobei mindestens eine Zwischenschicht eine biologisch aktive Substanz oder biologische Zellen zur Erzeugung einer biologisch aktiven Substanz enthält und die biologisch aktive Substanz ein parakriner Faktor, ein Hormon, ein Enzym, ein biochemischer Transmitter, oder Kombinationen davon ist,
- eine auf der obersten Zwischenschicht angebrachte Schicht M, die Mikrogelpartikel enthält, welche zumindest ein thermoresponsives Polymer umfassen.

2. Das Substrat gemäß Anspruch 1, wobei das thermoresponsive Polymer ein neutrales Polymer ist.

3. Das Substrat gemäß Anspruch 1 oder 2, wobei das thermoresponsive Polymer Monomereinheiten aufweist, die sich aus einem oder mehreren der folgenden Monomere ableiten:
- (Meth)Acrylsäureester,
- (Meth)Acrylsäureamid,
- einem vinylaromatischen Monomer,
- Vinylalkohol, der gegebenenfalls substituiert sein kann,
- Vinylether, der gegebenenfalls substituiert sein kann,
- Vinylamin, das gegebenenfalls substituiert sein kann,
- Vinylamid, das gegebenenfalls substituiert sein kann,
- Ethylenglycol, der gegebenenfalls substituiert sein kann,
- Propylenglycol, der gegebenenfalls substituiert sein kann,
- Ethylenimin, das gegebenenfalls substituiert sein kann,
- Oxazolin, das gegebenenfalls substituiert sein kann,
- C₅- und/oder C₆-Monosaccharide, wie z.B. D-Glucose, D-Mannose, D-Galactose, die gegebenenfalls substituiert sein können.

4. Das Substrat gemäß einem der vorstehenden Ansprüche, wobei das thermoresponsive Polymer aus einem der folgenden Polymere oder deren Gemischen ausgewählt wird:
(1) 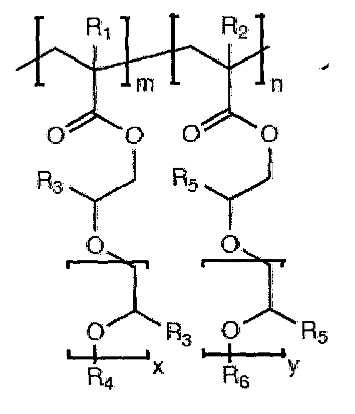 wobei
R₁, R₂ = H, Alkyl,
R₃, R₅ = H, Alkyl,
R₄, R₆ = H, Alkyl, Allyl, Phenyl, Benzyl, Acyl,
x und y unabhängig voneinander 1-50, bevorzugter 2-20 sind,
4≤ m + n ≤ 10⁷;
(2) 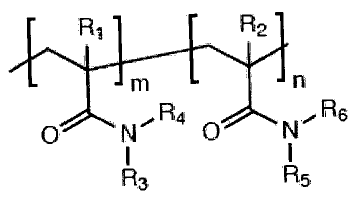 wobei
R₁, R₂ = H, Alkyl,
R₃, R₄, R₅, R₆ = H, Alkyl, Allyl, Phenyl, Benzyl,
4≤ m + n ≤ 10⁷;
(3) 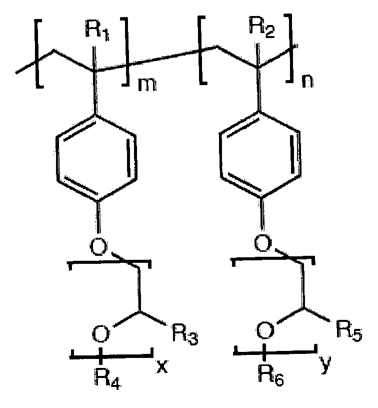 wobei
R₁, R₂ = H, Alkyl,
R₃, R₅ = H, Alkyl,
R₄, R₆ = H, Alkyl, Allyl, Phenyl, Benzyl, Acyl,
x und y unabhängig voneinander 1-50, bevorzugter 2-20 sind,
4≤ m + n ≤ 10⁷;
(4) 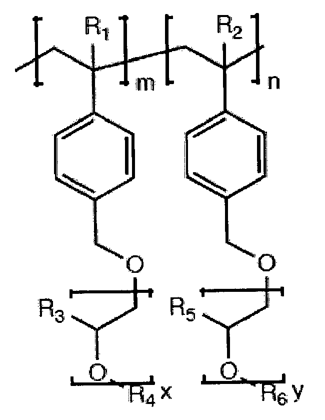 wobei
R₁, R₂ = H, Alkyl,
R₃, R₅ = H, Alkyl,
R₄, R₆ = H, Alkyl, Allyl, Phenyl, Benzyl, Acyl,
x und y unabhängig voneinander 1-50, bevorzugter 2-20 sind,
4≤ m + n ≤ 10⁷;
(5) 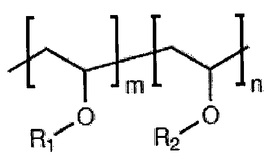 wobei
R₁, R₂ = H, Alkyl,
4≤m + n ≤ 10⁷;
(6) 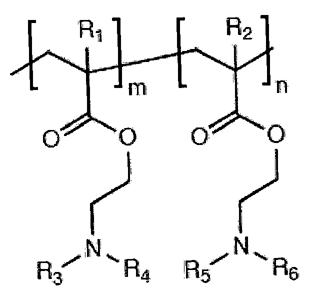 wobei
R₁, R₂ = H, Alkyl,
R₃, R₅ = H, Alkyl,
R₄, R₆ = H, Alkyl, Allyl, Phenyl, Benzyl, Acyl,
4≤ m + n ≤10⁷;
(7) 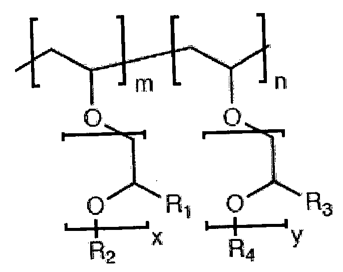 wobei
R₁, R₃ = H, Alkyl,
R2, R₄ = H, Alkyl, Allyl, Phenyl, Benzyl, Acyl,
x und y unabhängig voneinander 1-50, bevorzugter 2-20 sind,
4≤ m + n ≤ 10⁷;
(8) 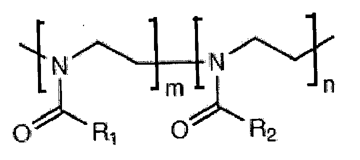 wobei
R₁, R₂ = H, Alkyl, Allyl, Phenyl, Benzyl,
4≤m+n≤10⁷;
(9) 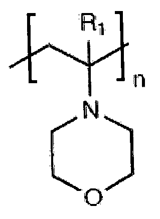 wobei
R₁, = H, Alkyl,
n > 5;
(10) 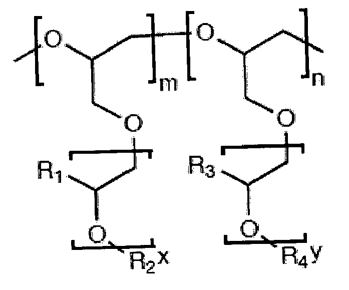 wobei
R₁, R₃ = H, Alkyl,
R₂, R₄ = H, Alkyl, Allyl, Phenyl, Benzyl, Acyl
x und y unabhängig voneinander 1-50, bevorzugter 2-20 sind,
4≤m + n≤10⁷;
(11) 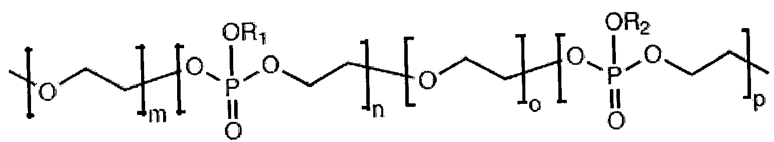 wobei
R₁, R₂ = H, Alkyl,
4≤n+n+o+p≤10⁷;
(12) 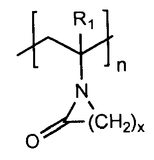 wobei
R₁ = H, Alkyl,
x 2 bis 6 ist,
n größer 5 ist, z.B. 6 ≤ n ≤ 10⁷
(13) 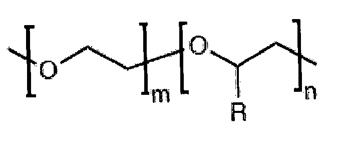 wobei
R = Alkyl,
10 > m : n > 0,1,
4 ≤ m + n ≤ 10⁷;
(14) 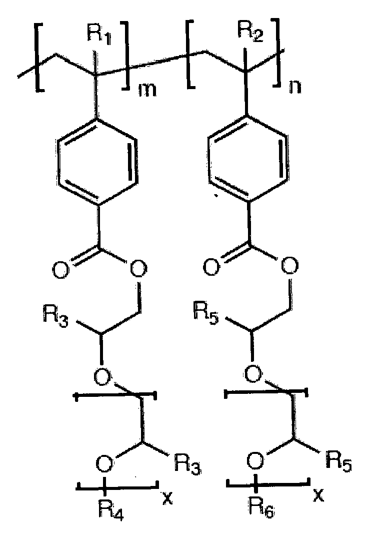 wobei
R₁, R₂ = H, Alkyl,
R₃, R₅ = H, Alkyl,
R₄, R₆ = H, Alkyl, Allyl, Phenyl, Benzyl, Acyl,
x und y unabhängig voneinander 1-50, bevorzugter 2-20 sind,
4 ≤ m + n ≤ 10⁷;
(15) 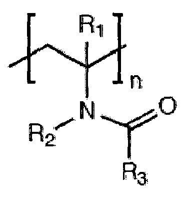 wobei
R₁, R₂ = H, Alkyl,
R₃ = Alkyl, Allyl, Phenyl, Benzyl,
4≤ n ≤ 10⁷;
(16) 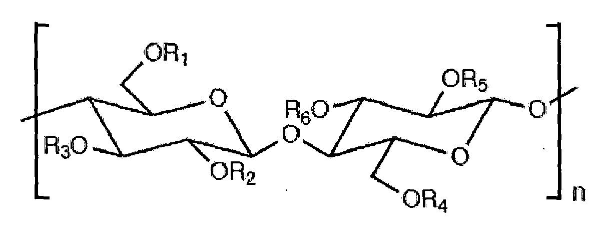 wobei
Ri, R₂, R₃, R₄, R₅, R₆ = H, Alkyl, Allyl, Phenyl, Benzyl, Acyl,
4≤n≤10⁷.

5. Das Substrat gemäß einem der vorstehenden Ansprüche, wobei die Mikrogelpartikel der Schicht M in Form einer Monolage, bevorzugt einer geschlossenen Monolage vorliegen.

6. Das Substrat gemäß Anspruch 5, wobei die Monolage eine Packungsdichte aufweist, die im Bereich von einer dichtesten Kugelpackung bis 0,15 Mikrogelpartikeln pro zweidimensionaler hexagonaler Elementarzelle liegt.

7. Das Substrat gemäß einem der vorstehenden Ansprüche, wobei das thermoresponsive Polymer einen Phasenübergang bei einer Temperatur T im Bereich von 10°C < T < 40°C aufweist.

8. Das Substrat gemäß einem der vorstehenden Ansprüche, wobei auf der die Mikrogelpartikel enthaltenden Schicht M biologisches Material, bevorzugt biologische Zellen, Proteine, Proteinaggregate, Mikroorganismen, oder Kombinationen davon, vorliegen.

9. Das Substrat gemäß einem der vorstehenden Ansprüche, wobei die biologisch aktive Substanz in der Zwischenschicht in Mikrogelpartikeln, Mikrokapseln, Vesikeln, Liposomen oder einem porösen Material oder Kombinationen davon vorliegt.

10. Das Substrat gemäß einem der vorstehenden Ansprüche, wobei das Substrat mindestens zwei Zwischenschichten Z1 und Z2 aufweist, und wobei die Zwischenschicht Z1 biologische Zellen enthält und die Zwischenschicht Z2 aus einem zellkompatiblen Material besteht.

11. Das Substrat gemäß einem der vorstehenden Ansprüche, wobei die Mikrogelpartikel der Schicht M über kovalente Bindungen mit der benachbarten Zwischenschicht verknüpft sind.

12. Ein Verfahren zur Herstellung eines Substrats nach einem der Ansprüche 1-11, umfassend:
- die Bereitstellung eines Substratkörpers mit einer Trägerfläche,
- Aufbringen einer oder mehrerer Zwischenschichten auf die Trägerfläche, wobei mindestens eine Zwischenschicht eine biologisch aktive Substanz oder biologische Zellen zur Erzeugung einer biologisch aktiven Substanz enthält und die biologisch aktive Substanz ein parakriner Faktor, ein Hormon, ein Enzym, ein biochemischer Transmitter, oder Kombinationen davon ist,
- Herstellung einer Dispersion von Mikrogelpartikeln, die ein thermoresponsives Polymer enthalten,
- Auftragen der Dispersion auf die oberste Zwischenschicht,
- Fixierung der Mikrogelpartikel auf der Zwischenschicht.

13. Ein Verfahren zur Kultivierung biologischer Zellen auf einem Substrat nach einem der Ansprüche 1-11, umfassend:
- Aufbringen von biologischen Zellen auf das Substrat,
- Einstellen von Kultivierungsbedingungen, so dass die biologischen Zellen einem Wachstum, einer Differenzierung und/oder einer Migration unterzogen werden.

14. Das Verfahren nach Anspruch 13, wobei das Substrat unter Bedingungen gehalten wird, so dass die biologisch aktive Substanz zu den biologischen Zellen auf dem Substrat diffundiert und ein Wachstum, eine Differenzierung und/oder eine Migration der Zellen auf dem Substrat bewirkt.

## Claims

1. Substrate, in particular for receiving biological cells, comprising:
- a substrate body that comprises a carrier surface,
- one or more intermediate layers Z which is/are applied to the carrier surface, wherein at least one intermediate layer contains a biologically active substance or biological cells for generating a biologically active substance and the biologically active substance is a paracrine factor, a hormone, an enzyme, a biochemical transmitter, or combinations thereof,
- a layer M that is applied to the uppermost intermediate layer and contains microgel particles which comprise at least one thermoresponsive polymer.

2. Substrate according to Claim 1, wherein the thermoresponsive polymer is a neutral polymer.

3. Substrate according to Claim 1 or 2, wherein the thermoresponsive polymer comprises monomer units that are derived from one or more of the following monomers:
- (meth)acrylic ester,
- (meth)acrylamide,
- a vinylaromatic monomer,
- vinyl alcohol, which can optionally be substituted,
- vinyl ether, which can optionally be substituted,
- vinylamine, which can optionally be substituted,
- vinylamide, which can optionally be substituted,
- ethylene glycol, which can optionally be substituted,
- propylene glycol, which can optionally be substituted,
- ethylenimine, which can optionally be substituted,
- oxazoline, which can optionally be substituted,
- C₅ and/or C₆ monosaccharides, such as, e.g., D-glucose, D-mannose, D-galactose, which can optionally be substituted.

4. Substrate according to any one of the preceding claims, wherein the thermoresponsive polymer is selected from one of the following polymers or mixtures thereof:
(1) 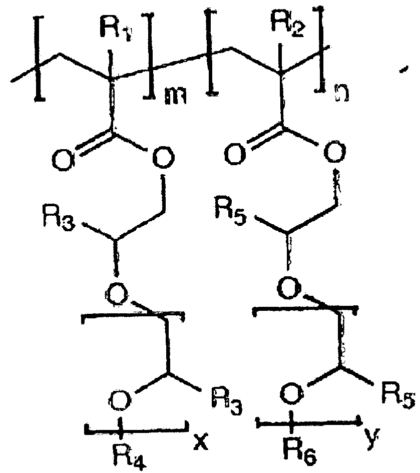 wherein
R₁, R₂ = H, alkyl,
R₃, R₅ = H, alkyl,
R₄, R₆ = H, alkyl, allyl, phenyl, benzyl, acyl,
x and y independently of one another are 1-50, more preferably 2-20,
4 ≤ m + n ≤ 10⁷;
(2) 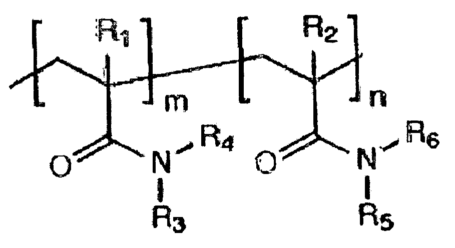 wherein
R₁, R₂ = H, alkyl,
R₃, R₄, R₅, R₆ = H, alkyl, allyl, phenyl, benzyl,
4 ≤ m + n ≤ 10⁷;
(3) 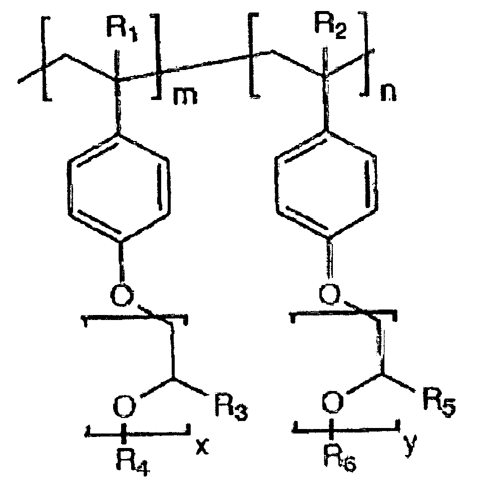 wherein
R₁, R₂ = H, alkyl,
R₃, R₅ = H, alkyl,
R₄, R₆ = H, alkyl, allyl, phenyl, benzyl, acyl,
x and y independently of one another are 1-50, more preferably 2-20,
4 ≤ m + n ≤ 10⁷;
(4) 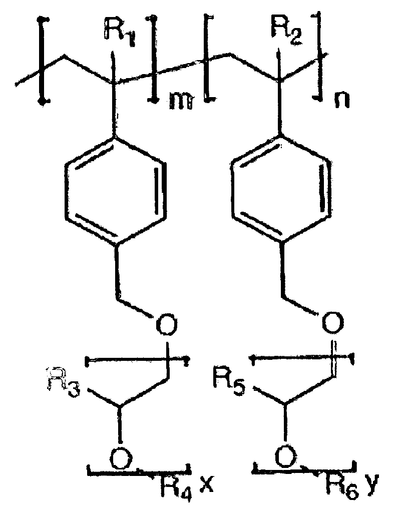 wherein
R₁, R₂ = H, alkyl,
R₃, R₅ = H, alkyl,
R₄, R₆ = H, alkyl, allyl, phenyl, benzyl, acyl,
x and y independently of one another are 1-50, more preferably 2-20,
4 ≤ m + n ≤ 10⁷;
(5) 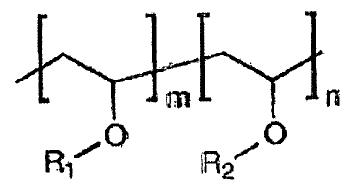 wherein
R₁, R₂ = H, alkyl,
4 ≤ m + n ≤ 10⁷;
(6) 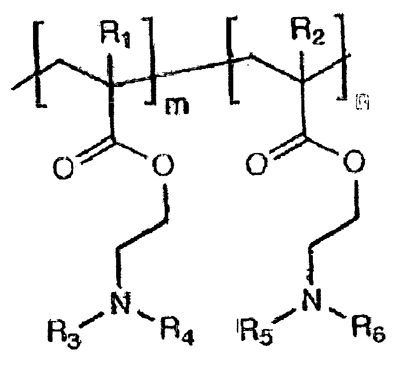 wherein
R₁, R₂ = H, alkyl,
R₃, R₅ = H, alkyl,
R₄, R₆ = H, alkyl, allyl, phenyl, benzyl, acyl,
4 ≤ m + n ≤ 10⁷;
(7) 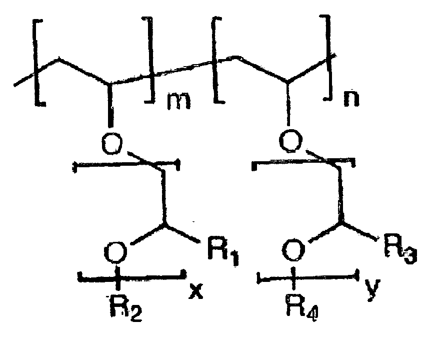 wherein
R₁, R₃ = H, alkyl,
R₂, R₄ = H, alkyl, allyl, phenyl, benzyl, acyl,
x and y independently of one another are 1-50, more preferably 2-20,
4 ≤ m + n ≤ 10⁷;
(8) 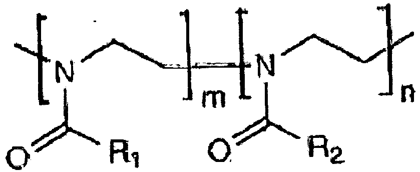 wherein
R₁, R₂ = H, alkyl, allyl, phenyl, benzyl,
4 ≤ m + n ≤ 10⁷;
(9) 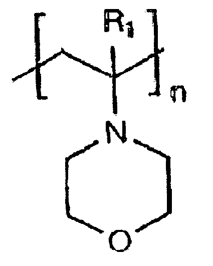 wherein
R₁ = H, alkyl,
n > 5,
(10) 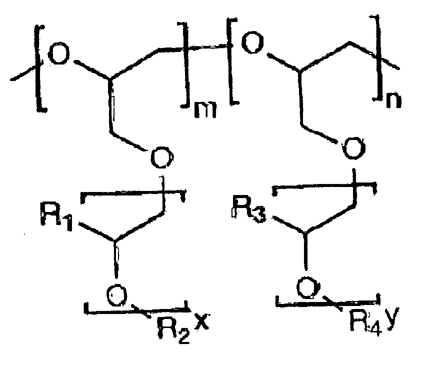 wherein
R₁, R₃ = H, alkyl,
R₂, R₄ = H, alkyl, allyl, phenyl, benzyl, acyl,
x and y independently of one another are 1-50, more preferably 2-20,
4 ≤ m + n ≤ 10⁷;
(11) 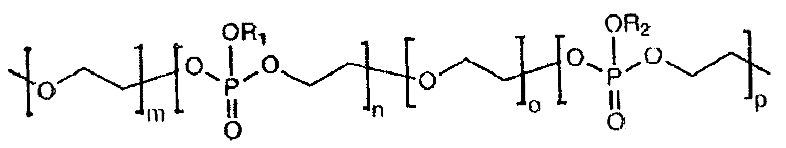 wherein
R₁, R₂ = H, alkyl,
4 ≤ m + n + o + p ≤ 10⁷;
(12) 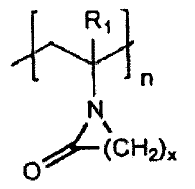 wherein
R₁ = H, alkyl,
x is 2 to 6,
n is greater than 5, e.g. 6 ≤ n ≤ 10⁷;
(13) 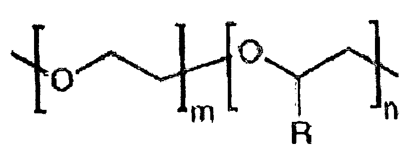 wherein
R = alkyl,
10 > m : n > 0.1,
4 ≤ m + n ≤ 10⁷;
(14) 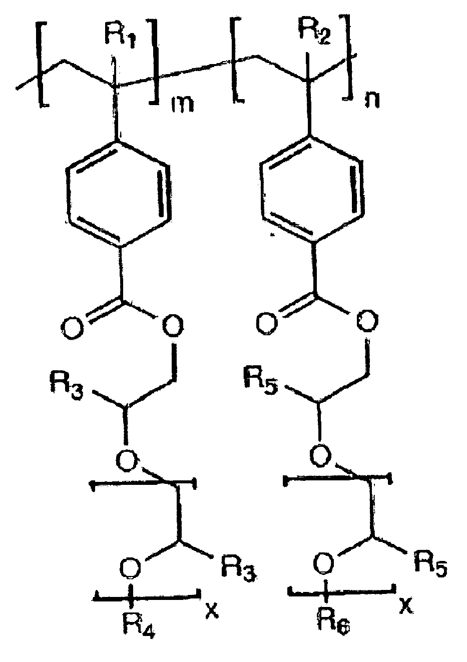 wherein
R₁, R₂ = H, alkyl,
R₃, R₅ = H, alkyl,
R₄, R₆ = H, alkyl, allyl, phenyl, benzyl, acyl,
x and y independently of one another are 1-50, more preferably 2-20,
4 ≤ m + n ≤ 10⁷ ;
(15) 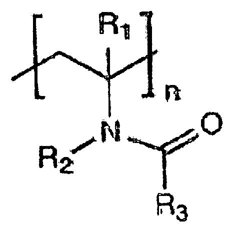 wherein
R₁, R₂ = H, alkyl,
R₃ = alkyl, allyl, phenyl, benzyl,
4 ≤ n ≤ 10⁷;
(16) 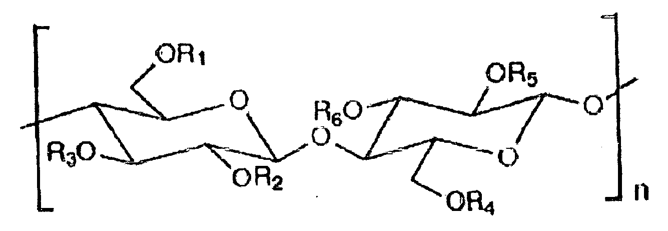 wherein
R₁, R₂, R₃, R₄, R₅, R₆ = H, alkyl, allyl, phenyl, benzyl, acyl,
4 ≤ n ≤ 10⁷.

5. Substrate according to any one of the preceding claims, wherein the microgel particles of the layer M are present in the form of a monolayer, preferably a closed monolayer.

6. Substrate according to Claim 5, wherein the monolayer has a packing density which is in the range from a tightest spherical packing to 0.15 microgel particles per two-dimensional hexagonal elemental cell.

7. Substrate according to any one of the preceding claims, wherein the thermoresponsive polymer has a phase transition at a temperature T in the range from 10°C < T < 40°C.

8. Substrate according to any one of the preceding claims, wherein biological material, preferably biological cells, proteins, protein aggregates, microorganisms, or combinations thereof are present on the layer M containing the microgel particles.

9. Substrate according to any one of the preceding claims, wherein the biologically active substance is present in the intermediate layer in microgel particles, microcapsules, vesicles, liposomes or a porous material or combinations thereof.

10. Substrate according to any one of the preceding claims, wherein the substrate has at least two intermediate layers Z1 and Z2, and wherein the intermediate layer Z1 contains biological cells, and the intermediate layer Z2 consists of a cell-compatible material.

11. Substrate according to any one of the preceding claims, wherein the microgel particles of the layer M are linked via covalent bonds to the neighbouring intermediate layer.

12. Method for producing a substrate according to any one of Claims 1-11, comprising:
- providing a substrate body having a carrier surface
- applying one or more intermediate layers to the carrier surface, wherein at least one intermediate layer contains a biologically active substance or biological cells for generating a biologically active substance, and the biologically active substance is a paracrine factor, a hormone, an enzyme, a biochemical transmitter, or combinations thereof,
- producing a dispersion of microgel particles that contain a thermoresponsive polymer,
- applying the dispersion to the uppermost intermediate layer,
- fixing the microgel particles on the intermediate layer.

13. Method for culturing biological cells on a substrate according to any one of Claims 1-11, comprising:
- applying biological cells to the substrate,
- establishing culturing conditions in such a manner that the biological cells undergo growth, differentiation and/or migration.

14. Method according to Claim 13, wherein the substrate is kept under conditions such that the biologically active substance diffuses to the biological cells on the substrate, and growth, differentiation and/or migration of the cells on the substrate is effected.

## Revendications

1. Substrat, en particulier destiné à recevoir des cellules biologiques, comprenant :
- un corps de substrat, qui présente une surface support,
- une ou plusieurs couches intermédiaires Z, qui est/sont appliquée(s) sur la surface support, au moins une couche intermédiaire contenant une substance biologiquement active ou des cellules biologiques pour produire une substance biologiquement active et la substance biologiquement active étant un facteur paracrine, une hormone, une enzyme, un transmetteur biochimique ou des combinaisons de ceux-ci,
- une couche M, appliquée sur la couche intermédiaire supérieure, qui contient des particules de microgel qui comprennent au moins un polymère thermosensible.

2. Substrat selon la revendication 1, le polymère thermosensible étant un polymère neutre.

3. Substrat selon la revendication 1 ou 2, le polymère thermosensible présentant des unités monomères qui sont dérivées d'un ou de plusieurs des monomères suivants :
- ester de l'acide (méth)acrylique,
- amide de l'acide (méth)acrylique,
- un monomère aromatique de vinyle,
- un alcool vinylique, qui peut le cas échéant être substitué,
- un vinyléther, qui peut le cas échéant être substitué,
- une vinylamine, qui peut le cas échéant être substituée,
- un vinylamide, qui peut le cas échéant être substitué,
- l'éthylèneglycol, qui peut le cas échéant être substitué,
- le propylèneglycol, qui peut le cas échéant être substitué,
- une éthylène-imine, qui peut le cas échéant être substituée,
- une oxazoline, qui peut le cas échéant être substituée,
- des monosaccharides en C₅ et/ou en C₆, tels que par exemple le D-glucose, le D-mannose, le D-galactose, qui peuvent le cas échéant être substitués.

4. Substrat selon l'une quelconque des revendications précédentes, le polymère thermosensible étant choisi parmi un des polymères suivants ou leurs mélanges :
(1) 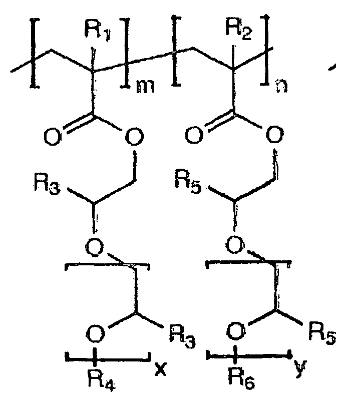 dans laquelle
R₁, R₂ = H, alkyle,
R₃, R₅ = H, alkyle,
R₄, R₆ = H, alkyle, allyle, phényle, benzyle, acyle,
x et y valent, indépendamment l'un de l'autre, 1-50, plus préférablement 2-20,
4 ≤ m + n ≤ 10⁷ ;
(2) 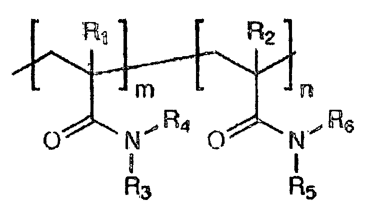 dans laquelle
R₁, R₂ = H, alkyle,
R₃, R₄, R₅, R₆ =H, alkyle, allyle, phényle, benzyle,
4 ≤ m + n ≤ 10⁷ ;
(3) 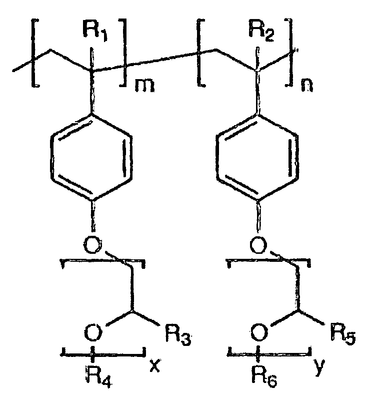 dans laquelle
R₁, R₂ = H, alkyle,
R₃, R₅ = H, alkyle,
R₄, R₆ = H, alkyle, allyle, phényle, benzyle, acyle,
x et y valent, indépendamment l'un de l'autre, 1-50, plus préférablement 2-20,
4 ≤ m + n ≤ 10⁷ ;
(4) 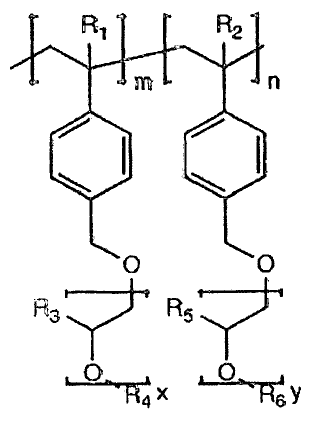 dans laquelle
R₁, R₂ = H, alkyle,
R₃, R₅ = H, alkyle,
R₄, R₆ = H, alkyle, allyle, phényle, benzyle, acyle,
x et y valent, indépendamment l'un de l'autre, 1-50, plus préférablement 2-20,
4 ≤ m + n ≤ 10⁷ ;
(5) 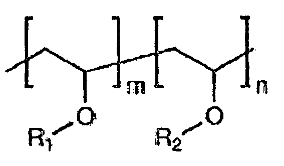 dans laquelle
R₁, R₂ = H, alkyle,
4 ≤ m + n ≤ 10⁷ ;
(6) 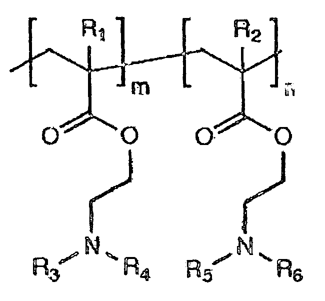 dans laquelle
R₁, R₂ = H, alkyle,
R₃, R₅ = H, alkyle,
R₄, R₆ = H, alkyle, allyle, phényle, benzyle, acyle,
4 ≤ m + n ≤ 10⁷ ;
(7) 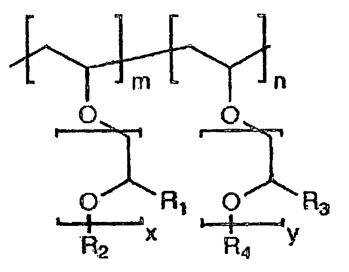 dans laquelle
R₁, R₃ = H, alkyle,
R₂, R₄ = H, alkyle, allyle, phényle, benzyle, acyle,
x et y valent, indépendamment l'un de l'autre, 1-50, plus préférablement 2-20,
4 ≤ m + n ≤ 10⁷;
(8) 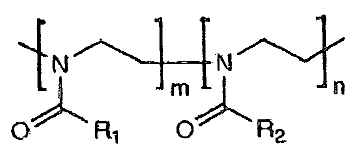 dans laquelle
R₁, R₂ = H, alkyle, allyle, phényle, benzyle,
4 ≤ m + n ≤ 10⁷ ;
(9) 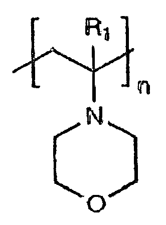 dans laquelle
R₁, = H, alkyle, n > 5 ;
(10) 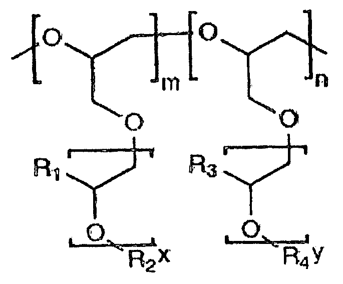 dans laquelle
R₁, R₃ = H, alkyle,
R₂, R₄ = H, alkyle, allyle, phényle, benzyle, acyle,
x et y valent, indépendamment l'un de l'autre, 1-50, plus préférablement 2-20,
4 ≤ m + n ≤ 10⁷;
(11) 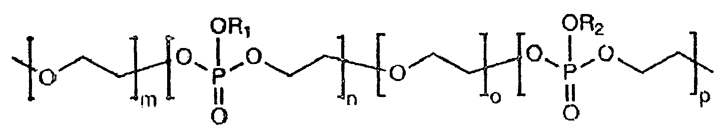 dans laquelle
R₁, R₂ = H, alkyle,
4 ≤ m + n + o + p ≤ 10⁷ ;
(12) 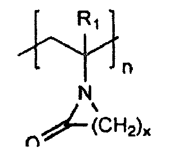 dans laquelle
R₁ = H, alkyle,
x vaut 2 à 6,
n est supérieur à 5, par exemple 6 ≤ n ≤ 10⁷ ;
(13) 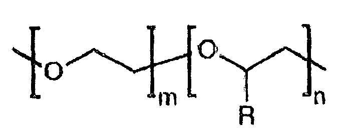 dans laquelle
R = alkyle,
10 > m:n > 0,1,
4 ≤ m + n ≤ 10⁷ ;
(14) 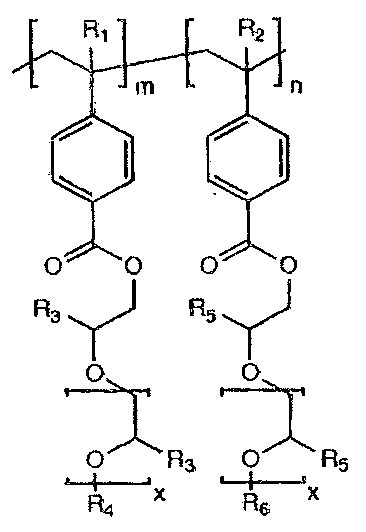 dans laquelle
R₁, R₂ = H, alkyle,
R₃, R₅ = H, alkyle,
R₄, R₆ = H, alkyle, allyle, phényle, benzyle, acyle,
x et y valent, indépendamment l'un de l'autre, 1-50, plus préférablement 2-20,
4 ≤ m + n ≤ 10⁷ ;
(15) 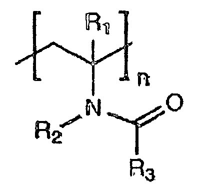 dans laquelle
R₁, R₂ = H, alkyle,
R₃ = alkyle, allyle, phényle, benzyle,
4 ≤ n ≤ 10⁷ ;
(16) 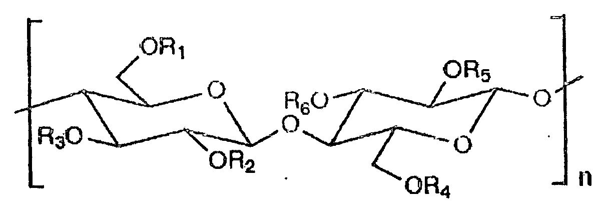 dans laquelle
R₁, R₂, R₃, R₄, R₅, R₆ = H, alkyle, allyle, phényle, benzyle, acyle,
4 ≤ n ≤ 10⁷.

5. Substrat selon l'une quelconque des revendications précédentes, les particules de microgel de la couche M se trouvant sous forme d'une monocouche, de préférence d'une monocouche fermée.

6. Substrat selon la revendication 5, la monocouche présentant une densité de garnissage qui se situe dans la plage d'un garnissage sous forme de billes le plus compact de jusqu'à 0,15 particule de microgel par cellule élémentaire hexagonale bidimensionnelle.

7. Substrat selon l'une quelconque des revendications précédentes, le polymère thermosensible présentant une transition de phase à une température T dans la plage de 10°C < T < 40°C.

8. Substrat selon l'une quelconque des revendications précédentes, une matière biologique, de préférence des cellules biologiques, des protéines, des agrégats de protéines, des micro-organismes ou leurs combinaisons se trouvant sur la couche M contenant les particules de microgel.

9. Substrat selon l'une quelconque des revendications précédentes, la substance biologiquement active dans la couche intermédiaire se trouvant dans des particules de microgel, des microcapsules, des vésicules, des liposomes ou un matériau poreux ou des combinaisons de ceux-ci.

10. Substrat selon l'une quelconque des revendications précédentes, le substrat présentant au moins deux couches intermédiaires Z1 et Z2 et la couche intermédiaire Z1 contenant des cellules biologiques et la couche intermédiaire Z2 étant constituée par un matériau compatible avec des cellules.

11. Substrat selon l'une quelconque des revendications précédentes, les particules de microgel de la couche M étant liées via des liaisons covalentes avec la couche intermédiaire adjacente.

12. Procédé pour la fabrication d'un substrat selon l'une quelconque des revendications 1-11, consistant à :
- préparer un corps de substrat présentant une surface support,
- appliquer une ou plusieurs couches intermédiaires sur la surface support, au moins une couche intermédiaire contenant une substance biologiquement active ou des cellules biologiques pour produire une substance biologiquement active et la substance biologiquement active étant un facteur paracrine, une hormone, une enzyme, un transmetteur biochimique ou des combinaisons de ceux-ci,
- préparer une dispersion de particules de microgel, qui contiennent un polymère thermosensible,
- appliquer la dispersion sur la couche intermédiaire supérieure,
- fixer les particules de microgel sur la couche intermédiaire.

13. Procédé pour la culture de cellules biologiques sur un substrat selon l'une quelconque des revendications 1-11, consistant à :
- appliquer des cellules biologiques sur le substrat,
- régler des conditions de culture de manière telle que les cellules biologiques sont soumises à une croissance, une différenciation et/ou une migration.

14. Procédé selon la revendication 13, le substrat étant maintenu dans des conditions telles que la substance biologiquement active diffuse vers les cellules biologiques sur le substrat et provoque une croissance, une différenciation et/ou une migration des cellules sur le substrat.
